# EUROPÄISCHE PATENTSCHRIFT

(11) **EP 2 567 681 B1**
(45) Veröffentlichungstag und Bekanntmachung des Hinweises auf die Patenterteilung: **12.11.2014**
(21) Anmeldenummer: 11007378.0
(22) Anmeldetag: 09.09.2011
(51) Int. Cl.: A61F 13/00, A61F 13/14, A61M 27/00, A61M 1/00

(54) **Wundauflage für den Abdominalbereich**
Wound dressing for the abdominal area
Pansement pour la zone abdominale

(43) Veröffentlichungstag der Anmeldung: 13.03.2013
(73) Patentinhaber: Paul Hartmann AG, 89522 Heidenheim (DE)
(72) Erfinder: Croizat, Pierre, Dr., 89542 Herbrechtingen (DE); Eckstein, Axel, Dr., 89522 Heldenheim (DE); Wolf, Cornelia, 89542 Herbrechtingen (DE)

(56) Entgegenhaltungen:
- WO-A1-03/057307
- WO-A1-2010/051068
- DE-A1-102008 062 472
- US-B1- 7 790 945

## Beschreibung

Die vorliegende Erfindung betrifft eine Wundauflage und eine Vorrichtung zur Verwendung bei der Unterdrucktherapie von Wunden im Abdominalbereich.

Vorrichtungen zur Unterdrucktherapie von Wunden und Verbände als Bestandteil derartiger Vorrichtungen sind im Stand der Technik bekannt.

So beschreibt beispielsweise die WO1993/009727 eine Vorrichtung zur Förderung der Wundheilung durch die Applikation von Unterdruck auf dem die Wunde aufweisenden und die Wunde umgebenden Hautbereich. Die Vorrichtung gemäß WO1993/009727 umfasst eine Vakuumeinrichtung zur Erzeugung des Unterdrucks, eine als Dichtungseinrichtung bezeichnete luftdichte Abdeckung der Wunde, welche mit der Vakuumeinrichtung in einer funktionellen Verbindung steht, sowie eine als Schutzeinrichtung bezeichnete Wundauflage zur Positionierung an der Wunde innerhalb der Dichtungseinrichtung. Bei der Schutzeinrichtung handelt es sich um einen porösen Polymerschaumstoff, beispielsweise um Polyester-Schaum. Ausweislich der Beschreibung von WO1993/009727 kann durch die Anwendung der Unterdrucktherapie die Wundheilung unterschiedlich Typen von Wunden wie beispielsweise Brandwunden, Druckwunden oder Platzwunden beschleunigt werden.

Der Ausdruck "Unterdruck" bezeichnet dabei einen innerhalb des Wundverbandes gegenüber dem Umgebungsluftdruck (atmosphärischer Luftdruck) erniedrigten Luftdruck. Unter "innerhalb des Wundverbandes" wird der durch das luftdichte Abdeckmaterial und dem Körpergewebe im Wundbereich gebildete Zwischenraum (Wundraum) verstanden. "Unterdruck" wird häufig auch als "negativer Druck" bezeichnet. Die Druckdifferenz zwischen dem Luftdruck innerhalb des Wundverbandes und dem Umgebungsluftdruck wird im Zusammenhang mit der Erfindung in mm Hg (Millimeter-Quecksilbersäule) angegeben, da dies im Bereich der Unterdrucktherapie üblich ist. 1 mm Hg entspricht einem Torr beziehungsweise 133,322 Pa (Pascal). Im Zusammenhang mit der Erfindung wird der Unterdruck, d.h. die Druckdifferenz zwischen dem Luftdruck innerhalb des Wundverbandes und dem Umgebungsluftdruck, als positiver Zahlenwert in mm Hg angegeben.

Besonders großflächige Wunden können im Abdominalbereich entweder infolge von Verletzungen oder infolge von chirurgischen Eingriffen entstehen. Chirurgische Eingriffe im Abdominalbereich werden beispielsweise bei der operativen Behandlung akuter und lebensbedrohlicher Erkrankungen des Bauchraums vorgenommen. Im Rahmen der postoperativen Versorgung derartiger chirurgischer Eingriffe kann auch die Notwendigkeit bestehen, den offenen Abdominalbereich mittels eines temporären Wundverschlusses nur vorübergehend abzudecken.

Freigelegte innere Organe oder das Omentum majus (auch als Bauchnetz oder großes Netz bezeichnet) bilden im Falle einer Abdominalwunde einen Wundgrund, also eine innerhalb eines Wundrandes befindliche Körperoberfläche. Bei der Versorgung einer Abdominalwunde wird eine zur Wunde hin orientierte Schicht der Wundauflage (im Folgenden als Wundkontaktschicht bezeichnet) direkt auf freigelegte innere Organe oder auf das Omentum majus aufgebracht. Die den freigelegten inneren Organen oder dem Omentum majus aufliegende Wundkontaktschicht dient bei der Unterdruck-Behandlung einer Abdominalwunde auch als Organschutzschicht, welche ein unerwünschtes Verkleben von Organen oder dem Omentum majus mit der Bauchdecke verhindern soll. Der Randbereich der Wundauflage wird üblicherweise in den durch Bauchdecke und innere Organe gebildeten Zwischenraum eingeführt.

Während der Behandlung einer Wunde am offenen Abdomen kann es erforderlich sein, eine sehr große Flüssigkeitsmenge, beispielsweise bis zu 5 l innerhalb von 48 Stunden, abzuleiten. Sehr große abzuleitende Flüssigkeitsmengen können z.B. während der operativen Behandlung eines Darmverschlusses (lleus) oder einer Bauchfellentzündung (Peritonitis) entstehen.

Aus der WO01/85248 ist ein Verband zur temporären Abdeckung von Wunden aus Unfällen oder chirurgischen Eingriffen, insbesondere von Abdominalwunden, bekannt. Der Verband ist zur Verwendung in der Unterdrucktherapie vorgesehen. Die WO01/85248 schlägt vor, den Wundgrund mit einer mit Löchern versehenen Folie abzudecken. Auf die Folie, welche die Wundkontaktschicht darstellt, wird ein poröser Schaum aufgebracht. Auf der von der Wunde abgewandten Seite umfasst der Verband einen für Flüssigkeiten undurchlässigen Abdeckfilm mit Kleberand zum luftdichten Verschließen des Wundbereichs. Weiterhin sind Verbindungsmittel vorgesehen, welche sich durch den Abdeckfilm hindurch bis zum porösen Schaum erstrecken, um den Wundraum mit einer Unterdruckquelle verbinden zu können. Im Betrieb kann Wundexsudat aus dem Wundraum entfernt werden, indem die Flüssigkeit durch die Öffnungen der gelochten Folie zunächst zum porösen Schaum und weiterhin über den Schaum zum Verbindungsmittel gelangt, welches sich im direkten Kontakt mit dem porösen Schaum befindet.

Die WO01/85248 zielt insbesondere auf einen Wundverband, welcher ohne Beschädigung bzw. Traumatisierung der Wunde gewechselt werden kann. Derartige Beschädigungen beim Verbandwechsel können entstehen, wenn während der Therapie Verklebungen zwischen dem Wundverband und dem darunter liegendem Gewebe gebildet werden oder wenn Gewebe in den Verband einwächst, insbesondere beim Einwachsen faserigen Gewebes in den Schaumstoffanteil des Wundverbandes. Die WO01/85248 möchte das unerwünschte Einwachsen von Gewebe in den Verband vermindern, indem der direkte Kontakt porösen Materials mit dem Wundgrund vermindert wird. Die WO01/85248 schlägt dazu vor, dass die Fläche der Öffnungen der mit Löchern versehenen Folie weniger als 10 % der effektiven Fläche der Folie beträgt. Idealerweise sollte die offene Fläche weniger als 1 oder 2 % der Fläche der Folie betragen. Gemäß der WO01/85248 können die Öffnungen in der Folie in Form von Schlitzen vorliegen, was den Kontakt zwischen Schaum und Wundgrund weiter vermindert.

Die WO2010/051068 beschreibt eine Wundauflage für das offene Abdomen, welche eine Vielzahl umhüllter strangartiger Druckverteilungs-Elemente umfasst, die durch ein zentrales Verbindungselement funktionell gekoppelt sind. Auf der von der Wunde abgewandten Seite des zentralen Verbindungselementes ist ein weiteres Druckverteilungs-Element aufgebracht. Bei den Druckverteilungs-Elementen kann es sich beispielsweise um einen offenzelligen Schaumstoff handeln. Die Wundauflage ist zuschneidbar.

Die US2009/0099519 beschreibt einen Abdominalverband für die Unterdrucktherapie, welcher ein umhülltes Polster und ein Heizelement zur Temperaturkontrolle umfasst.

Die WO2010/124844 offenbart eine Wundauflage, welche insbesondere zum Einsatz am offenen Abdomen vorgesehen ist. Die Wundauflage umfasst zwei bahnförmige Elemente, welche einen zwischen ihren inneren Begrenzungsflächen liegenden Drainageraum bilden. Mindestens eines der bahnförmigen Elemente kann Öffnungen aufweisen, wobei insbesondere in den Drainageraum mündende kanalförmige Öffnungen vorgesehen sind, welche eine Kapillarwirkung begünstigen können.

Die Patentanmeldung DE102010052336A1 des Anmelders der vorliegenden Patentanmeldung beschreibt einen Verband zur Verwendung bei der Unterdrucktherapie von Wunden, insbesondere von Wunden im Abdominalbereich, umfassend eine flexible Folie als Wundkontaktschicht und mindestens ein auf die Folie aufgebrachtes Leitungsmittel, wobei sowohl das Leitungsmittel als auch die Folie eine Vielzahl von Öffnungen aufweisen, so dass eine Fluid-Verbindung zwischen dem Leitungsmittel und dem Wundraum herstellbar ist. Das auf der Folie aufgebrachte Leitungsmittel weist dabei neben den endständigen Öffnungen eine Vielzahl von seitlichen Öffnungen auf, die eine Aufnahme von Wundfluid in den durchgehenden Hohlraum ermöglichen.

Bei der Drainage großflächiger und stark exsudierender Wunden im Abdominalbereich ergibt sich insbesondere das Erfordernis, eine konstante und gleichmäßige Verteilung der Drainagekapazität über die gesamte Fläche der Wunde zu gewährleisten. Insbesondere soll eine zu den Rändern der Wundauflage hin nachlassende Drainageleistung vermieden werden, da ansonsten eine unerwünschte Ansammlung von Flüssigkeiten im peripheren Bereich des Abdomens auftreten kann.

Bei der Applikation einer Abdominalwundauflage auf freigelegte innere Organe oder das Omentum majus muss der Anwender, beispielsweise ein Chirurg, auf eine gleichmäßige Auflage des Verbandes auf den Wundgrund achten. Insbesondere kann es für den Anwender schwierig sein, den umlaufenden Randbereich der Wundauflage zwischen Bauchdecke und innere Organe zu applizieren.

Aufgabe der vorliegenden Erfindung ist es, eine verbesserte Abdominalwundauflage für die Unterdrucktherapie zur Verfügung zu stellen. Insbesondere ist es Aufgabe der vorliegenden Erfindung, eine Abdominalwundauflage für die Unterdrucktherapie zur Verfügung zu stellen, welche eine erhöhte Drainagekapazität und eine verbesserte Anwendungsfreundlichkeit aufweist. Die Abdominalwundauflage sollte dabei über eine Drainagekapazität verfügen, welche eine gleichmäßige Ableitung von Wundexsudat über die gesamte Fläche der Wunde einschließlich der peripheren Kavitäten des Abdomens während der gesamten Dauer der Unterdrucktherapie gewährleistet. Die Wundauflage soll leicht anzulegen, gut an die Gegebenheiten der individuellen Wunde anpassbar und ohne Verklebungen bzw. Verwachsungen mit dem Untergrund zu wechseln sein.

Diese Aufgabe wird erfindungsgemäß gelöst durch eine Wundauflage zur Verwendung bei der Unterdrucktherapie abdominaler Wunden, nach Anspruch 1. Die Wundauflage umfasst eine erste flexible Folie mit einer ersten und einer zweiten Seite, wobei die erste Seite zum Aufbringen auf den Wundgrund, insbesondere auf freigelegte innere Organe oder auf das Omentum majus, vorgesehen ist, und wobei die erste Folie weiterhin eine Vielzahl von über die Fläche verteilten Öffnungen aufweist. Des Weiteren umfasst die Wundauflage mindestens drei auf der zweiten Seite der ersten Folie aufgebrachte Leitungsmittelabschnitte aus einem biegsamen elastomeren Material mit einer Dickenerstreckung (H) von höchstens 20 mm, wobei jeder der Leitungsmitteiabschnitte mindestens einen durchgehenden Hohlraum aufweist, und wobei jeder der Leitungsmittelabschnitte flach ausgebildet ist. Weiterhin zeichnet sich die Wundauflage dadurch aus, dass jeder der Leitungsmitteiabschnitte mit Ausnahme der endständigen Öffnungen über keine weiteren Öffnungen verfügt. Auf der im Gebrauch von der Wunde abgewandten Seite der Wundauflage ist mindestens eine zur Mitte der Wundauflage hin offene Tasche vorhanden, welche das gleichmäßige Aufbringen und Auslegen der Wundauflage auf den Wundgrund erleichtert. Bei der Anwendung in der Unterdrucktherapie von Wunden ist die Wundauflage mit einer Unterdruckquelle Fluid-leitend verbindbar, so dass eine Fluid-Kommunikation zwischen der Unterdruckquelle und dem Wundraum herstellbar ist.

Bei der Dickenerstreckung (H) handelt es sich um die maximale Erstreckung eines auf einer ersten Folie aufgebrachten Leitungsmittelabschnittes in einer senkrecht zur Ebene der ersten Folie verlaufenden Dimension.

Der Abdeckfilm wird üblicherweise auf dem die Wunde umgebenden Hautbereich dichtend befestigt. Unter Fluid werden in diesem Zusammenhang sowohl Flüssigkeiten, beispielsweise Wundfluid, Blut, Darmflüssigkeit oder Spülflüssigkeit, als auch Gase, beispielsweise Luft oder Kohlendioxid, verstanden.

Gegenüber den aus dem Stand der Technik bekannten Wundauflagen stellt die erfindungsgemäße Wundauflage weitere Hohlräume in Form von mindestens drei biegsamen und flach ausgebildeten Leitungsmittelabschnitts zur Unterstützung der Ableitung von Wundexsudat zur Verfügung. Dadurch kann die Drainagekapazität der Wundauflage insbesondere in den peripheren Bereichen wesentlich erhöht und weiterhin über die Dauer der Anwendung stabilisiert werden. Die erfindungsgemäße Wundauflage gewährleistet eine kontinuierliche und störungsfreie Ableitung von Wundfluid über die Dauer der Anwendung der Vorrichtung, beispielsweise über einen Zeitraum von 24 bis 72 Stunden. Eine kontinuierliche und störungsfreie Ableitung von Wundfluid aus den peripheren Bereichen der Wunde wird dabei insbesondere über die Lumina der mindestens drei Leitungsmittelabschnitte gewährleistet, da diese, beispielsweise im Vergleich zu einer Schaumlage, aufgrund ihrer offenen Querschnittsfläche weniger anfällig für Verstopfungen sind.

Die biegsam und flach ausgebildeten Leitungsmittelabschnitte gewährleisten sowohl eine Ableitung von Wundfluid aus den peripheren Bereichen des Abdomens, als auch eine kontinuierliche und störungsfreie Applikation von Unterdruck (d.h. die Herstellung eines gegenüber dem Umgebungsluftdruck erniedrigten Luftdrucks) im Wundraum über die Dauer der therapeutischen Anwendung. Ein Druckabfall im peripheren Bereich des Abdomens, also ein nachlassender Unterdruck im Randbereich des Wundraums, wird vermieden.

Die erfindungsgemäße Wundauflage kann anwenderseitig, also seitens des Arztes oder des medizinischen Personals, uneingeschränkt an die Form und Größe der Wunde angepasst werden, beispielsweise durch einfaches Zuschneiden mit einer sterilen Schere. Insofern es dagegen erwünscht ist, das Produkt bereits herstellerseitig an vordefinierte Wundformen und Wundgrößen angepasst bereit zu stellen, so ergeben sich durch den Aufbau der erfindungsgemäßen Wundauflage in dieser Hinsicht gleichfalls keine Einschränkungen.

Die erfindungsgemäße Wundauflage umfasst eine erste flexible Folie mit einer ersten und einer zweiten Seite. Im Gebrauch wird die flexible Folie mit ihrer ersten Seite in direktem Kontakt auf den Wundgrund, also insbesondere auf die chirurgisch oder verletzungsbedingt freigelegten inneren Organe oder auf das Omentum majus, gelegt. Es ist daher wesentlich, dass die erste flexible Folie aus einem Material besteht, welches während der Dauer der Anwendung nicht mit den freigelegten inneren Organen, dem Omentum majus oder der Bauchdecke verklebt oder verwächst. Das Material soll atraumatische Eigenschaften aufweisen.

Geeignete Materialien für die erste flexible Folie umfassen thermoplastische Folien, insbesondere Folien aus Ethylvinylacetat (im Folgenden: EVA), Polyurethan (im Folgenden: PU), Polyethylen (im Folgenden: PE), Polyethylenterephthalat (im Folgenden: PET), PTFE (im Folgenden: Polytetrafluorethylen), Polyvinylchlorid (im Folgenden: PVC), thermoplastische Elastomere (im Folgenden: TPE), Polyorganosiloxan (im Folgenden: Silikon) oder einer Mischung daraus. Unter der Bezeichung TPE sind in diesem Zusammenhang thermoplastische Elastomere auf Olefinbasis (TPO), vernetzte thermoplastische Elastomere auf Olefinbasis (TPV), thermoplastische Elastomere auf Urethanbasis (TPU), thermoplastische Polyesterelastomere bzw. thermoplastische Copolyester (TPC), Styrol-Blockcopolymere (TPS) und thermoplastische Copolyamide (TPA) umfasst. Vorzugsweise handelt es sich bei der thermoplastischen Folie um eine PE-Folie.

Das Flächengewicht der ersten Folie sollte mindestens 30 g / m² und höchstens 150 g /m², vorzugsweise mindestens 45 g / m² und höchstens 95 g / m² und insbesondere mindestens 55 g /m² und höchstens 65 g /m² betragen.

Gemäß einer besonders bevorzugten Ausführungsform handelt es sich bei der ersten flexiblen Folie um eine Stützfilm-freie PE-Folie mit einem Flächengewicht von 55 g /m² bis 65 g /m² auf, wobei das Flächengewicht nach der Norm EN ISO 2286 - 2 bestimmt wird, beispielsweise die Produkte "Folie MEDIFOL^{®} 3D, Type 44600" oder "Folie MEDIFOL^{®} 3D T16, Type 44601 T16" der Firma rkw Prolife (Wasserburg, Deutschland).

Die erste flexible Folie weist eine Vielzahl von Öffnungen auf, welche einen Durchtritt von Wundfluid ermöglichen. Bei den Öffnungen kann es sich um Löcher oder um Schlitze handeln. Als Loch wird in diesem Zusammenhang eine Öffnung bezeichnet, welche in der Aufsicht im ungedehnten Zustand eine offene Fläche aufweist. Als Schlitz wird in diesem Zusammenhang eine Öffnung bezeichnet, welche in der Aufsicht im ungedehnten Zustand keine offene Fläche aufweist. Hinsichtlich Form und Größe der Öffnungen kann in geeigneter Weise auf die erwünschte Durchlässigkeit der Folie für Wundfluid abgestellt werden. Denkbar sind runde, ovale, eckige oder beispielsweise sternförmige Löcher. Bei den Schlitzen kann es sich beispielsweise um längliche oder kreuzförmige Schlitze handeln.

Die Öffnungen können gleichmäßig, d.h. in regelmäßig sich wiederholenden Mustern oder zufällig über die Fläche der ersten Folie verteilt vorliegen. Es ist dabei auch möglich, dass nur ein erster Anteil der ersten flexiblen Folie eine Vielzahl von über die Fläche verteilten Öffnungen aufweist, während ein weiterer Anteil der ersten flexiblen Folie keine Öffnungen aufweist. So kann es beispielsweise vorteilhaft sein, dass ein zentraler Anteil der ersten Folie eine Vielzahl von Öffnungen aufweist, während ein peripherer Anteil der Folie keine Öffnungen aufweist. Eine umgekehrte Anordnung ist gleichfalls möglich.

Falls es sich bei den Öffnungen um Schlitze handelt, sollten diese eine Länge von jeweils mindestens 1 mm und höchstens 30 mm, vorzugsweise mindestens 2 mm und höchstens 20 mm und insbesondere mindestens 5 mm und höchstens 10 mm aufweisen. Dabei hat sich gezeigt, dass eine Folie, welche Schlitze mit einer Länge von mindestens 5 mm und höchstens 10 mm aufweist, wobei zwischen 10 und 90 derartiger Schlitze auf einer FolienFläche von 100 cm² eingebracht werden, besonders vorteilhafte Eigenschaften hinsichtlich Stabilität bei gleichzeitig ausreichender Fluid-Durchlässigkeit aufweist.

Vorzugsweise weist die erste flexible Folie eine Vielzahl von Öffnungen auf, wobei es sich bei den Öffnungen um Löcher handelt. Der Durchmesser der Löcher kann hinsichtlich der erwünschten Fluid-Durchlässigkeit in geeigneter Weise angepasst werden, beispielsweise bei kreisförmigen Löchern in einem Bereich vom 0,1 mm bis 5 mm. Bereiche zwischen mindestens 0,2 mm und höchstens 0,4 mm sind dabei hinsichtlich einer günstigen Kombination von Fluid-Durchlässigkeit und atraumatischen Eigenschaften der Folie besonders bevorzugt. Löcher mit einem Durchmesser von 0,3 mm weisen hierbei besonders günstige Eigenschaften auf.

Falls es sich bei den Öffnungen in der ersten Folie um Löcher handelt, soll die Summe der offenen Fläche der Löcher des Weiteren mindestens 0,5 %, vorzugsweise jedoch mindestens 10 % der Flächenerstreckung der Folie betragen, um eine ausreichende Durchlässigkeit der Folie für Wundfluid zu gewährleisten. Dabei soll möglichst eine offene Fläche von 25 % der Folie nicht überschritten werden, da sich dies andernfalls negativ auf die Stabilität der Folie auswirken könnte. Vorzugsweise beträgt die Summe der offenen Fläche der in der Folie vorhandenen Löcher deshalb mindestens 0,5 % und höchstens 25 % der Flächenerstreckung, vorzugsweise mindestens 12 % und höchstens 23 % der Flächenerstreckung.

Fluid-Durchlässigkeit, Stabilität und die Neigung von Gewebe zum Durchwachsen der Folie werden durch die offene Fläche der Folie beeinflusst.

Gemäß einer bevorzugten Ausführungsform der Erfindung beträgt die offene Fläche der in der ersten Folie vorhandenen Löcher mindestens 13 % und höchstens 15 % der Flächenerstreckung der Folie. Eine derartige Folie erweist sich insbesondere in Bezug auf Fluid-Durchlässigkeit, atraumatische Eigenschaften und Stabilität als vorteilhaft. Die Anzahl der in der ersten Folie pro Fläche vorhandenen Öffnungen sollte bei dieser Ausführungsform mindestens 150 pro cm² und höchstens 190 pro cm², insbesondere mindestens 165 pro cm² und höchstens 171 pro cm², betragen.

Gemäß einer weiteren bevorzugten Ausführungsform der Erfindung beträgt die offene Fläche der in der ersten Folie vorhandenen Löcher mindestens 20 % und höchstens 22 % der Flächenerstreckung der Folie. Eine derartige Folie weist eine hohe Fluid-Durchlässigkeit und Flexibilität bzw. Weichheit auf. Die Anzahl der in der ersten Folie pro Fläche vorhandenen Öffnungen sollte bei dieser weiteren Ausführungsform mindestens 260 pro cm² und höchstens 300 pro cm², insbesondere mindestens 275 pro cm² und höchstens 285 pro cm², betragen.

Denkbar ist auch, dass die erste Folie eine Vielzahl von über die Fläche verteilten Öffnungen aufweist, wobei es sich bei den Öffnungen um eine Kombination von Löchern und Schlitzen handelt. Dies kann beispielsweise derart ausgeführt werden, dass ein bestimmter Flächenbereich der ersten Folie Löcher aufweist und ein anderer Flächenbereich der ersten Folie Schlitze aufweist.

Erfindungsgemäß sind hinsichtlich Form, Größe, Anzahl und Anordnung der Öffnungen in der ersten flexiblen Folie weitere, hier nicht speziell aufgeführte Varianten umfasst.

Die Öffnungen werden üblicherweise vor dem Befestigen der mindestens drei Leitungsmittelabschnitte in die erste Folie eingebracht oder es wird eine Folie als Ausgangsmaterial verwendet, welche bereits herstellerseitig eingebrachte Öffnungen aufweist. Es ist jedoch auch möglich die Öffnungen erst nach dem Aufbringen der Leitungsmittelabschnitte zu erzeugen.

Gemäß einer besonders bevorzugten Ausführungsform der Erfindung werden die in der ersten Folie vorhandenen Öffnungen durch Perforieren oder Stanzen der Folie derart erzeugt, dass weitestgehend konisch oder zylindrisch geformte Öffnungen entstehen, die eine dreidimensionale Struktur aufweisen, so dass die Folie infolgedessen eine glatte Seite und eine der glatten Seite gegenüberliegenden aufgeraute Seite aufweist. Das Einbringen von Öffnungen mit einer dreidimensionalen Struktur kann beispielsweise durch eine Stanzwalze erfolgen. Alternativ können Öffnungen mit einer dreidimensionalen Struktur bereits während der Herstellung der Folie erzeugt werden, beispielsweise durch Führung der extrudierten, noch schmelzflüssigen Folie über eine rotierende Unterdruck-Lochwalze. Besondere Vorteile bei Verwendung einer ersten Folie mit derartigen dreidimensional strukturierten Öffnungen für den erfindungsgemäßen Wundverband ergeben sich insbesondere dann, wenn die erste, zum Aufbringen auf den Wundgrund vorgesehene Seite der ersten Folie durch die glatte Seite der Folie gebildet wird und die zweite Seite der ersten Folie durch die aufgeraute Seite der Folie gebildet wird. Die zum Aufbringen auf den Wundgrund vorgesehene glatte Seite der ersten Folie weist eine geringe Neigung zum Verkleben mit dem Wundgrund auf. Anderseits bewirken die dreidimensional strukturierten Öffnungen eine Beabstandung des Wundgrundes zu weiteren Lagen des Verbandes, beispielsweise zu einer Schaumstofflage, falls derartige zusätzliche Verbandschichten vorhanden sind. Eine Beabstandung zu weiteren Lagen kann ein unerwünschtes Einwachsen faserigen Gewebes in weitere Verbandlagen vermindern.

Folien, welche konisch oder zylindrisch geformte Öffnungen aufweisen, sind kommerziell erhältlich, beispielsweise die vorgenannten Folien mit den Bezeichnungen "Folie MEDIFOL^{®} 3D, Type 44600" oder "Folie MEDIFOL^{®} 3D T16, Type 44601 T16" (Hersteller rkw ProLife, Wasserburg, Deutschland).

Auf die zweite Seite der ersten Folie werden mindestens drei Leitungsmittelabschnitte aus einem biegsamen elastomeren Material mit einer Dickenerstreckung (H) von höchstens 20 mm aufgebracht, wobei jeder der Leitungsmittelabschnitte mindestens einen durchgehenden Hohlraum aufweist, und wobei jeder der Leitungsmittelabschnitte flach ausgebildet ist. Dabei verfügt jeder der Leitungsmittelabschnitte mit Ausnahme der endständigen Öffnungen über keine weiteren Öffnungen.

Der Verbund aus erster Folie und Leitungsmittelabschnitten soll insgesamt über eine ausreichende Flexibilität und Weichheit verfügen, so dass sich die Wundauflage dem Wundgrund dicht anschmiegen kann. Flexibilität und Weichheit des Verbundes aus erster Folie und Leitungsmittelabschnitten werden durch die jeweiligen Eigenschaften der Einzelkomponenten Folie und Leitungsmittel bestimmt. Daher ist es wesentlich, dass sowohl die erste Folie, als auch die auf die erste Folie aufgebrachten Leitungsmittelabschnitte aus einem biegsamen elastomeren Material bestehen. Gemäß einer bevorzugten Ausführungsform sollte jeder der Leitungsmittelabschnitte deshalb aus einem Material gefertigt sein, welches eine Shore A Härte von höchstens 70 aufweist (bestimmt nach DIN 53505 vom August 2000, und zwar bei 23°C an einem wie in der Norm beschrieben plattenförmigen ebenen und glatten Probekörper einer Dicke von 6 mm). In besonders vorteilhafter Weise werden für einen Leitungsmittelabschnitt noch weichere Materialien, das heißt Materialien mit einer Shore A Härte von weniger als 65, insbesondere mit einer Shore A Härte von weniger als 62, besonders bevorzugt mit einer Shore A Härte von 60 eingesetzt. Flexibilität und Weichheit von erster Folie und Leitungsmittelabschnitt erleichtern zudem die Zuschneidbarkeit zur Anpassung der Wundauflage an die Wundform.

Bei den mindestens drei Leitungsmittelabschnitten handelt es sich vorzugsweise um einen Abschnitt eines ein- oder mehriumigen Drainageschlauches, wobei der Drainageschlauch beispielsweise EVA, PU, PVC, PE, PET, PTFE, TPE, Silikon oder eine Mischung daraus umfasst. Ein im Rahmen der Erfindung geeigneter Leitungsmittelabschnitt kann durch Ablängen aus einem längeren Drainageschlauch gewonnen werden.

Weiterhin ist es hinsichtlich der Flexibilität des Verbundes aus erster Folie und Leitungsmittelabschnitten wesentlich, dass jeder der mindestens drei Leitungsmittelabschnitte eine Dickenerstreckung (H) von höchstens 20 mm, vorzugsweise weniger als 15 mm, insbesondere von weniger als 7 mm, aufweist.

Die mindestens drei auf die erste Folie aufgebrachten Leitungsmittelabschnitte sind flach ausgebildet, d.h. sie weisen hinsichtlich ihres äußeren Umfangs vorzugsweise eine insgesamt flache Form auf. Unter einer insgesamt flachen Form eines Leitungsmittelabschnittes wird im Zusammenhang mit der Erfindung verstanden, dass ein Leitungsmittelabschnitt ein Verhältnis seiner Querausdehnung bzw. Breite B (maximale Erstreckung des Querschnittes des Leitungsmittelabschnittes in einer senkrecht zur Ebene der ersten Folie verlaufenden Dimension) zu seiner Dickenerstreckung bzw. Höhe H (maximale Erstreckung des auf einer ersten Folie aufgebrachten Leitungsmittelabschnittes in einer senkrecht zur Ebene der ersten Folie verlaufenden Dimension) von mehr als 1,25, vorzugsweise mehr als 1,5, insbesondere mehr als 3 aufweist, wobei die Dickenerstreckung (H) höchstens 20 mm, vorzugsweise weniger als 15 mm, insbesondere weniger als 7 mm beträgt. Die insgesamt flache Form der mindestens drei Leitungsmittelabschnitte ist vorteilhaft, um eine im Gebrauch stabile und gegebenenfalls unlösbare Verbindung mit der ersten Folie zu gewährleisten. Weiterhin erweist sich die insgesamt flache Form der Leitungsmittelabschnitte als vorteilhaft beim Auflegen der Wundauflage und beim Tragen der Wundauflage während der Unterdrucktherapie.

Gemäß einer vorteilhaften Ausführungsform der Erfindung beträgt die Breite B des Leitungsmittelabschnittes dabei zwischen 8 mm und 40 mm, insbesondere zwischen 15 und 35 mm. Gemäß einer besonders bevorzugten Ausführungsform der Erfindung weist der Leitungsmittelabschnitt eine Breite B von 18 bis 22 mm und eine Dickenerstreckung H von 5 bis 6 mm auf, wobei der Leitungsmittelabschnitt aus einem Material mit einer Shore A Härte von 60 gefertigt ist.

Die mindestens drei Leitungsmittelabschnitte werden erfindungsgemäß auf die zweite Seite der ersten Folie aufgebracht. Unter "aufgebracht" wird im Zusammenhang mit der vorliegenden Erfindung verstanden, dass a) ein Leitungsmittelabschnitt mit der zweiten Seite der ersten Folie unlösbar und flächenhaft verbunden wird und/oder b) dass ein Leitungsmittelabschnitt auf der zweiten Seite der ersten Folie durch Befestigungsmittel an der Folie gehalten wird.

Im Falle der ersten Möglichkeit a) soll ein Leitungsmittelabschnitt dabei insbesondere mit wenigstens 10 %, vorzugsweise wenigstens 25 %, insbesondere mindestens 50 %, seiner senkrecht auf die erste Folie projizierten Fläche mit der zweiten Seite der ersten Folie unlösbar und flächenhaft verbunden werden. Unter "unlösbar verbunden" wird in diesem Zusammenhang verstanden, dass sich ein Leitungsmittelabschnitt während des normalen therapeutischen Einsatzes der Wundauflage nicht von der ersten Folie löst. Eine in diesem vorgenannten Sinne unlösbare Verbindung der Leitungsmittelabschnitte mit der ersten Folie kann beispielsweise durch Verkleben, Verschweißen oder durch die Verwendung eines doppelseitigen Klebebandes erreicht werden. Besonders bevorzugt handelt es sich bei dem Leitungsmittelabschnitt um einen Drainageschlauch mit einem insgesamt im Wesentlichen rechteckigem äußerem Umfang, bei welchem mindestens 50 % seiner senkrecht auf die erste Folie projizierten Fläche mit der zweiten Seite der ersten Folie unlösbar und flächenhaft verbunden werden kann.

Im Falle der zweiten Möglichkeit b) soll ein Leitungsmittelabschnitt durch Befestigungsmittel auf der ersten Folie gehalten wird. Unter Befestigungsmittel wird dabei eine Vielzahl dem Fachmann bekannter Befestigungsmittel verstanden, beispielsweise Klebestreifen oder Klammern. Bei dem Befestigungsmittel kann es sich auch um eine zweite Folie handeln, welche mit der zweiten Seite der ersten Folie verbunden ist und einen Leitungsmittelabschnitt taschenartig zwischen erster und zweiter Folie halten kann.

Ein Leitungsmittelabschnitt weist ein Lumen in Form einer oder mehrerer durchgehender Hohlräume auf, um eine Ableitung von Wundfluid zur Unterdruckquelle hin, sowie einen Unterdruck im Wundraum zu gewährleisten. Entsprechend kann ein Leitungsmitteiabschnitt entweder nur einen Hohlraum, oder aber mehrere Hohlräume umfassen, um das Lumen zu bilden.

Falls ein Leitungsmittelabschnitt jeweils mehrere Hohlräume aufweist, so können die einzelnen Hohlräume des Leitungsmittelabschnittes über Öffnungen miteinander verbunden sein, um ein kontinuierliches Lumen zu bilden. Es ist jedoch auch denkbar, dass ein Leitungsmittelabschnitt mehrere durchgehend verlaufende Hohlräume aufweist, welche nicht miteinander in Verbindung stehen. Dabei sollte die gesamte offene Querschnittsfläche des Lumens mindestens 1 mm², vorzugsweise mindestens 5 mm², betragen, um eine ausreichende Durchflusskapazität für die Ableitung von Wundfluid bereit zu stellen. Gemäß einer besonders bevorzugten Ausfühungsform soll das Lumen jedes einzelnen Leitungsmitteiabschnittes eine Querschnittsfläche von insgesamt 10 bis 50 mm², vorzugsweise 20 bis 35 mm² aufweisen. Unter "gesamter offener Querschnittsfläche des Lumens" wird in diesem Zusammenhang die Summe der offenen Querschnittsflächen der einzelnen Hohlräume eines einzelnen Leitungsmittelabschnittes verstanden, wobei sich der Leitungsmittelabschnitt in einem nicht-zusammengedrückten Zustand befindet. Vorzugsweise umfasst ein im Rahmen der Erfindung verwendbarer Leitungsmittelabschnitt zwei oder drei durchgehende Hohlräume, welche insbesondere miteinander Fluid-leitend verbunden sind.

Gemäß einer weiteren besonders bevorzugten Ausführungsform weist jeder der mindestens drei Leitungsmittelabschnitte nur einen einzigen durchgehenden Hohlraum auf.

Dabei ist wesentlich, dass jeder der Leitungsmittelabschnitte über eine ausreichende Unterdruckstabilität verfügen. Unter einer ausreichenden Unterdruckstabilität des Leitungsmittelabschnittes wird in Zusammenhang mit der Erfindung verstanden, dass der Leitungsmittelabschnitt beim Anlegen von Unterdruck nicht vollständig kollabiert, so dass ein Fluid-Fluß durch den Leitungsmittelabschnitt möglich ist. Insbesondere wird unter einer ausreichenden Unterdruckstabilität verstanden, dass der Leitungsmittelabschnitt bei einem in der Unterdrucktherapie eingesetzten Unterdruck von typischerweise 125 mmHg einen offenen Querschnitt von mindestens 1 mm² behält. Ein unerwünschtes Zusammendrücken des Leitungsmittels im Unterdruck kann auf unterschiedliche, dem Fachmann bekannte Weise, verhindert werden, beispielsweise durch eine geeignete Querschnittsgeometrie und/oder durch Auswahl geeigneter Materialien. So können im Leitungsmittelabschnitt beispielsweise asymmetrisch angeordnete Innenwandrippen vorgesehen sein, welche sich vorteilhaft auf die Unterdruckstabilität des Leitungsmittelabschnittes auswirken. Erfindungsgemäß geeignete Leitungsmittel mit einer ausreichenden Unterdruckstabilität sind auch als Fertigprodukt in für medizinische Anwendungen geeigneter Qualität kommerziell erhältlich. Derartige Leitungsmittel werden üblicherweise als Drainageschläuche bezeichnet.

Gemäß einer besonders vorteilhaften Ausführungsform weist der Leitungsmittelabschnitt eine Beschichtung oder Imprägnierung mit einer antikoagulierend wirkenden Substanz (beispielsweise Heparin, oder eine andere, üblicherweise zur Beschichtung von medizinischen Schläuchen oder Röhrchen verwendete Substanz mit antikoagulierender Wirkung) auf. Mittels einer derartigen antikoagulierenden Beschichtung kann einer Verstopfung des Leitungsmittelabschnittes durch Blutklumpen vorgebeugt werden, so dass ein gleichmäßig hoher Durchfluss von Wundexsudat über eine längere Anwendungsdauer gewährleistet bleibt. Mit einem Antikoagulans beschichtete medizinische Drainageschläuche sind als Fertigprodukt kommerziell erhältlich, beispielsweise von der Firma Axiom Medical (Bürstadt, Deutschland).

Die mindestens drei auf der ersten Folie aufgebrachten Leitungsmittelabschnitte befinden sich auf der von der Wunde abgewandten zweiten Seite der ersten Folie. Vorzugsweise werden die mindestens drei Leitungsmittelabschnitte in einer kreuz- oder sternförmigen Anordnung auf die erste Folie aufgebracht. Dabei ist es möglich, dass die mindestens drei kreuz- oder sternförmig auf der ersten Folie angeordneten Leitungsmittelabschnitte im Zentrum der ersten Folie aneinander stoßen. Jedoch kann es hinsichtlich einer Erhöhung der Drainagekapazität der Wundauflage vorteilhafter sein, wenn die mindestens drei kreuz- oder sternförmig auf der ersten Folie angeordneten Leitungsmittelabschnitte im Zentrum der ersten Folie nicht aneinander stoßen. Idealerweise werden die Leitungsmittelabschnitte derart angeordnet, dass die zum Zentrum der ersten Folie hin weisenden Enden einen Abstand b von 0,5 bis 7 cm zu den jeweils benachbart angeordneten Enden aufweisen. Bei einer kreuz- oder sternförmigen Anordnung einer geraden Anzahl von auf der ersten Folie angeordneten Leitungsmittelabschnitten sollte der Abstand a gegenüberliegender Enden der Leitungsmittelabschnitten vorzugsweise 1 bis 12 cm betragen.

Die mindestens drei, vorzugsweise mindestens vier Leitungsmittelabschnitte erstrecken sich dabei vom zentralen Bereich der ersten Folie bis zum peripheren Bereich der ersten Folie, insbesondere bis zum Rand der ersten Folie. Es kann jedoch im Zusammenhang mit einer alternativen Ausführungsform vorteilhaft sein, dass sich die Leitungsmittelabschnitte nicht bis zum Rand der ersten Folie erstrecken, so dass ein Randbereich der ersten Folie vorhanden ist, welcher keine aufgebrachten Leitungsmittelabschnitte aufweist. Typischerweise wird nämlich der Randbereich der Wundauflage nach dem Auflegen auf den Wundgrund vorsichtig zwischen die Bauchdecke und den Wundgrund eingeschoben. Ein wie vorstehend beschriebener Randbereich der ersten Folie, auf welchen keine Leitungsmittelabschnitte aufgebracht sind, wäre dann bei dieser alternativen Ausführungsform insbesondere zum Einschieben zwischen die Bauchdecke und den Wundgrund vorgesehen.

Gemäß einer sehr vorteilhaften Ausführungsform der Erfindung umfasst die Wundauflage eine zweite flexible Folie, wobei die zweite Folie zum Aufbringen auf die im Gebrauch von der Wunde abgewandte Seite des Verbundes aus erster Folie und den mindestens drei Leitungsmittelabschnitten vorgesehen ist. Vorzugsweise ist die zweite Folie aus einem weitestgehend Fluid-undurchlässigem Material gefertigt. Zur Herstellung der zweiten flexiblen Folie können insbesondere die vorstehend bereits im Zusammenhang mit der ersten flexiblen Folie erwähnten Materialien und Fertigprodukte verwendet werden, beispielsweise thermoplastische Folien, insbesondere Folien aus EVA, PU, PE, PET, PTFE, PVC, TPE, Silikon oder einer Mischung daraus.

Die zweite Folie wird unlösbar auf dem Verbund aus erster Folie und Leitungsmittelabschnitten befestigt, beispielsweise durch Verkleben oder durch Verschweißen. Als Befestigungsstellen kommen dabei entweder die Leitungsmittelabschnitte und/oder diejenigen Bereiche der ersten flexiblen Folie in Frage, welche zwischen den Leitungsmittelabschnitten lokalisiert sind.

Eine auf den Verbund aus erster Folie und Leitungsmittelabschnitten aufgebrachte zweite flexible Folie kann dazu beitragen, die Stabilität und Handhabbarkeit der Wundauflage weiter zu verbessern. Durch eine geeignete Auswahl von erster Folie und zweiter Folie kann die Durchlässigkeit der Wundauflage für Wundfluid, sowie die atraumatischen Eigenschaften der Wundauflage an die jeweiligen therapeutischen Erfordernisse angepasst werden.

Gemäß einer ersten vorteilhaften Ausführungsform ist die zweite Folie weitestgehend Fluid-undurchlässig ausgebildet und weist lediglich eine einzige, vorzugsweise im zentralen Bereich angeordnete Öffnung auf.

Gemäß einer weiteren Ausführungsform der Wundauflage weist die zweite Folie eine Vielzahl von über die Fläche verteilten Öffnungen auf, welche zum Durchleiten von Fluid geeignet sind. Dabei kann es sich als vorteilhaft erweisen, wenn die in der ersten Folie vorhandenen Öffnungen und die in der zweiten Folie vorhandenen Öffnungen so angeordnet sind, dass die Öffnungen weitestgehend nicht zueinander in Deckung stehen. Unter "weitestgehend nicht zueinander in Deckung stehend" wird in diesem Zusammenhang verstanden, dass zueinander in Deckung stehende Öffnungen von erster und zweiter Folie höchstens zufällig und nur in geringer Anzahl vorhanden sind. Insbesondere sollen dabei mindestens 90% vorzugsweise 95% der Öffnungen in der ersten Folie nicht mit einer Öffnung in der zweiten Folie in Deckung stehen.

Die zweite Folie kann auf unterschiedliche Weise auf dem Verbund aus erster Folie und Leitungsmittelabschnitten angeordnet sein. Beispielhafte Ausführungsformen werden im Folgenden dargelegt, wobei weitere Anordnungen denkbar und von der Erfindung umfasst sind.

Gemäß einer ersten Ausführungsform wird die zweite Folie auf die mindestens drei Leitungsmittelabschnitte geklebt, so dass ein stabiler Verbund aus erster Folie, Leitungsmittelabschnitten und zweiter Folie entsteht. Dies ist technisch leicht zu realisieren, beispielsweise indem jeder der Leitungsmittelabschnitte an seiner Oberfläche mit einem Kleber beschichtet wird und dann die zweite flexible Folie aufgelegt wird.

Gemäß einer weiteren Ausführungsform ist die zweite Folie mit der ersten Folie direkt verklebt, jedoch nicht mit dem Leitungsmittelabschnitt. Die Verklebung von erster Folie und zweiter Folie kann dabei flächig, punktförmig oder linienförmig erfolgen. Es ist auch denkbar, dass erste Folie und zweite Folie nicht verklebt, sondern durch Verschweißen miteinander in eine unlösbare Verbindung gebracht werden. Das Schweißen kann dabei insbesondere punktförmig oder entlang von Schweißnähten erfolgen. Eine direkte, unlösbare Verbindung von erster und zweiter Folie ergibt eine besonders stabile Anordnung.

Gemäß einer weiteren Ausführungsform ist es auch möglich, die zweite Folie mit der ersten Folie und mit dem Leitungsmittelabschnitt zu verkleben. Auf diese Weise ergibt sich ein besonders stabiler Verbund.

Gemäß einer besonders vorteilhaften Ausführungsform erfolgt die Befestigung der zweiten Folie auf der ersten Folie durch punktförmiges Verkleben oder Verschweißen der Folien, wobei eine Vielzahl von über die Fläche verteilten Haftpunkten vorgesehen ist. Bei einer derartigen Verbindung von erster und zweiter Folie entsteht ein Fluid-leitender Zwischenraum zwischen den Folien, welcher den Abfluss von Wundexsudat in vorteilhafter Weise begünstigen kann. Bei der hier genannten besonders vorteilhaften Ausgestaltung der Erfindung befinden sich die Leitungsmittelabschnitte in einer Tasche zwischen den beiden Folien, und werden auf diese Weise auf der ersten Folie gehalten. Die Verschweißung von erster und zweiter Folie dient hierbei als Befestigungsmittel für die Leitungsmittelabschnitte, so dass darauf verzichtet werden kann die Leitungsmittelabschnitte mit der ersten Folie zu verkleben.

Zur Aufrechterhaltung der Drainagekapazität der Wundauflage während der Unterdruckbehandlung wird im Zusammenhang mit einer weiteren vorteilhaften Ausgestaltung der Erfindung vorgeschlagen, die vor der Wundauflage umfassten Folienmaterialien mit einer anti-koagulierend wirkenden Substanz zu versehen. Hiermit kann gegebenenfalls eine Verstopfung der Öffnungen oder- falls vorhanden - des durch erste und zweite Folie gebildeten Zwischenraumes reduziert werden. Gemäß einer besonders vorteilhaften Ausführungsform weist das Folienmaterial eine Beschichtung oder Imprägnierung mit einer antikoagulierend wirkenden Substanz (beispielsweise Heparin, oder eine andere, üblicherweise zur Beschichtung von medizinischen Oberflächen oder Röhrchen verwendete Substanz mit antikoagulierender Wirkung) auf.

Gemäß einer besonders vorteilhaften Ausgestaltung der Erfindung umfasst die Wundauflage ein Verbindungselement, welches auf die zweite Seite der ersten flexiblen Folie aufgebracht werden kann und an welchem die Endabschnitte der mindestens drei Leitungsmittelabschnitte unlösbar befestigt werden können. Das Verbindungselement wird, vorzugsweise im mittigen Bereich der ersten Folie, aufgelegt oder unlösbar befestigt. Das Verbindungselement umfasst zweckmäßigerweise einen Kunststoff, beispielsweise aus EVA. PU, PVC, PE, PET, PTFE, TPE, Silikon oder eine Mischung daraus. Dabei ist es hinsichtlich der Anwendung vorteilhaft, wenn das Verbindungselement insgesamt weich und flexibel ausgebildet ist. Vorzugsweise sollte das Verbindungselement aus einem elastomeren Material bestehen, welches eine Shore A Härte von höchstens 80, vorzugsweise höchstens 70 und insbesondere von höchstens 60 aufweist.

Das Verbindungselement erleichtert die korrekte Positionierung der mindestens drei Leitungsmittelabschnitte im Sinne einer Auflageplatte.

Gemäß einer ersten möglichen Ausführungsform werden die Enden oder Endbereiche der Leitungsmittelabschnitte am Verbindungselement im Sinne einer Auflageplatte derart befestigt, dass die Leitungsmittelabschnitte voneinander getrennt vorliegen. Dies erfolgt demnach so, dass die die Enden Leitungsmittelabschnitte nicht in Berührung zueinander stehen. Entsprechend wird durch das Verbindungselement kein die vorhandenen Leitungsmittelabschnitte Fluid-leitend verbindender kontinuierlicher Hohlraum gebildet. Vorzugsweise werden mindestens drei Leitungsmittelabschnitte auf dem Verbindungselement befestigt, wobei die mindestens drei Leitungsmittelabschnitte in einer kreuz- oder sternförmigen Anordnung vorliegen.

Gemäß einer zweiten denkbaren Ausführungsform bildet das Verbindungselement einen zentralen Hohlraum in Sinne einer flachen Dose, wobei der zentrale Hohlraum mit den Lumina der Leitungsmittelabschnitte über ein offenes Ende Fluid-leitend verbunden ist. Der Hohlraum des Verbindungselement weist auf der von der Wunde abgewandten Seite Durchgänge auf, um eine Fluid-Kommunikation mit dem Unterdruckanschlussstück herstellen zu können. Die Leitungsplatte kann rund, quadratisch oder polygonal ausgebildet sein und weist einen Durchmesser von mindestes 1 cm und höchstens 12 cm, insbesondere mindestens 2 cm und höchstens 10 cm auf. Vorzugsweise werden mindestens drei Leitungsmittelabschnitte dichtend mit dem Hohlraum der flachen Dose verbunden, wobei die mindestens drei Leitungsmittelabschnitte in einer kreuz- oder sternförmigen Anordnung vorliegen.

Bei der Anwendung der Wundauflage zur Abdeckung großflächiger Wunden des Abdominalbereichs wird üblicherweise der Randbereich der ersten Folie oder der Randbereich des Verbundes aus erster Folie, Leitungsmittelabschnitten und - falls vorhanden - zweiter Folie, zwischen die Bauchdecke und den Wundgrund oder auf das Omentum majus eingebracht. Dazu wird typischerweise ein flaches chirurgisches Instrument, beispielsweise ein Bauch- oder Darmspatel, verwendet. Die Applikation des Randbereichs der Wundauflage zwischen Bauchdecke und den Wundgrund stellt große technische Anforderung an den Chirurgen und geht mit einer Verletzungsgefahr der inneren Organe einher. Dabei hat es sich insbesondere als schwierig erwiesen, den Randbereich der Wundauflage faltenfrei unter die Bauchdecke zu applizieren. Es wurde nun gefunden, dass die erfindungsgemäße Wundauflage wesentlich sicherer, schneller und gleichzeitig weitgehend faltenfrei in den Bereich zwischen innere Organe (oder Omentus majus) und Bauchdecke eingebracht werden kann. Hierzu weist die Wundauflage auf der im Gebrauch von der Wunde abgewandten Seite mindestens eine vorwiegend zur Mitte der Wundauflage hin offene Tasche auf. Der Anwender kann dazu ein flaches chirurgisches Instrument, beispielsweise einen Bauch- und Darmspatel, in die Tasche einführen und sodann die durch die Tasche vorübergehend am Spatel gehaltene Wundauflage vorsichtig unter die Bauchdecke einschieben. Nach dem Einschieben des Randbereichs der Wundauflage unter die Bauchdecke wird der Spatel wieder aus der Tasche herausgezogen. Vorzugsweise umfasst die Wundauflage eine Vielzahl von Taschen. Es können dann nacheinander oder gleichzeitig mehrere periphere Anteile der Wundauflage unter die Bauchdecke eingebracht werden, insofern auf der Wundauflage entsprechend angeordnete Taschen vorgesehen sind. Anstelle eines Instrumentes kann der Anwender zum Applizieren der Wundauflage auch einen oder mehrere Finger in die mindestens eine Tasche einführen.

Erfindungsgemäß umfasst die Wundauflage deshalb mindestens eine vorwiegend zur Mitte der Wundauflage hin offene Tasche, welche das gleichmäßige Aufbringen und Auslegen der Wundauflage auf den Wundgrund erleichtert. Die mindestens eine Tasche ist dabei vorzugsweise randständig auf der Wundauflage angeordnet. Unter randständig wird hier verstanden, dass die Tasche überwiegend im peripheren Anteil der Wundauflage angebracht wird. Der Abstand des äußeren Randes der Tasche zum Rand der Wundauflage könnte bei einer in diesem Sinne als randständigen bezeichneter Anordnung auf einer kreisförmigen Wundauflage mit einem Durchmesser von 45 cm beispielsweise 0 cm bis 10 cm betragen. Die Tiefe der mindestens einen Tasche beträgt vorzugsweise höchstens 15 cm, insbesondere höchstens 10 cm. Die mindestens eine Tasche ist zum Aufbringen auf die im Gebrauch von der Wunde abgewandte Seite (wundabseitig) derjenigen Folienlage vorgesehen, welche im Gebrauch von der Wunde abgewandt vorliegt: Insofern die Wundauflage lediglich eine erste flexible Folie umfasst, ist die Tasche zum Aufbringen auf die zweite Seite der ersten Folie vorgesehen. Insofern die Wundauflage lediglich aus zwei Folienlagen besteht, d.h. aus erster flexibler Folie und zweiter flexibler Folie, ist die

Tasche im Allgemeinen zum Aufbringen auf die zweite Seite der zweiten Folie vorgesehen. Eine Ausnahme kann sich hierbei ergeben, wenn die zweite Folie nur einen Anteil der ersten Folie überfängt. In diesem Falle wäre es auch möglich die Tasche auf einem nicht von der zweiten Folie bedeckten Anteil der ersten Folie anzubringen. Insofern die Wundauflage eine oder mehrere weitere flexible Folienlagen umfasst, ist die Tasche im allgemeinen zum Aufbringen auf die wundabseitige Seite der am weitesten wundabseitig orientierten Folienlage vorgesehen. Dies bedeutet, dass die Tasche stets wundabseitig auf der Wundauflage vorgesehen ist.

Im Rahmen der Erfindung geeignete Taschen sind in einer Vielzahl von Formen vorstellbar. Wesentlich ist dabei, dass eine vorwiegend zur Mitte der Wundauflage hin orientierte Öffnung vorhanden ist, in welche das chirurgische Instrument, insbesondere ein Bauch- und Darmspatel, eingeschoben bzw. eingehakt werden kann. Weiterhin ist wesentlich, dass eine der Öffnung gegenüberliegende Seite der Tasche dem eingeschoben bzw. eingehakten chirurgischen Instrument einen Widerhalt zu geben vermag. Am einfachsten ist ein derartiger Widerhalt durch eine der Öffnung der Tasche gegenüberliegende Naht zu realisieren. Die Öffnung der Tasche sollte vorwiegend zur Mitte der Wundauflage hin orientiert sein, damit ein vom Zentrum der Wundauflage her nach außen bewegtes chirurgisches Instrument in die Öffnung eingeschoben werden kann und dort einen Widerhalt zu finden vermag.

Eine vorwiegend zur Mitte der Wundauflage hin orientiert Öffnung der Tasche kann gleichermaßen eine manuelle Applikation der Wundauflage erleichtern, falls der Anwender anstelle eines chirurgischen Instrumentes einen Finger in die mindestens eine Tasche einführen möchte.

Die Tasche sollte hinsichtlich ihrer Form und Größe auf das chirurgische Instrument oder gegebenenfalls auf einen menschlichen Finger abgestimmt sein. Die Tasche kann beispielsweise im Wesentlichen die Form eines Rechtecks, eines Trapez, eines Halbrunds, eines Dreiecks, eines Kreisrings oder eines Kreisringsektors aufweisen, wobei weitere hier nicht näher beschriebene Formen, die sich für den Fachmann aus der Haltefunktion der Tasche ergeben, umfasst sind.

Die Tiefe der Tasche, also der Abstand der Öffnung der Tasche bis zum gegenüberliegenden Rand der Tasche, sollte vorzugsweise höchstens 15 cm betragen, insbesondere höchstens 10 cm. Die Tiefe sollte mindestens 0,3 cm, vorzugsweise jedoch mindestens 1 cm betragen, damit das in die Tasche eingeführte chirurgische Instrument oder ein gegebenenfalls in die Tasche eingeführter Finger beim Applizieren der Wundauflage nicht aus der Tasche herausrutschen kann. Eine erfindungsgemäß geeignete Tasche weist deshalb vorzugsweise eine Tiefe von 1 cm bis 10 cm auf.

Hinsichtlich der Breite b der Tasche ist es gleichfalls erforderlich, diese auf das bei der Applikation der Wundauflage zu Anwendung kommende chirurgische Instrument abzustimmen, weshalb die Breite b der Tasche mindestens der Breite des Instruments aufweisen muss, da dieses andernfalls nicht in die Tasche eingeführt werden kann. Es ist jedoch vorteilhaft, die Tasche wesentlich breiter als das Instrument auszulegen, da bei einer solchermaßen gestalteten Tasche das Einführen des Instrumentes in die Tasche einfacher bewerkstelligt werden kann. Der Anwender muss in diesem Falle nicht darauf achten, eine vergleichsweise enge Öffnung der Tasche zu finden, was sich im Falle einer stark blutenden Wunde als schwierig erweisen kann. Die Breite b der Tasche beträgt im Allgemeinen mindestens 1 cm, vorzugsweise mindestens 2 cm.

Erfindungsgemäß umfasst die Wundauflage mindestens eine auf der im Gebrauch von der Wunde abgewandten Seite der Wundauflage (wundabseits) vorhandene Tasche. Es erweist sich jedoch als besonders vorteilhaft für die Applikation der Wundauflage, wenn die Wundauflage eine Vielzahl von Taschen umfasst, welche an verschiedenen Stellen der Wundauflage angebracht sind. Hierbei wird vorgeschlagen dass die Wundauflage mindestens 3 Taschen umfasst, vorzugsweise mindestens 4, insbesondere mindestens 6 Taschen. Die Taschen sollten in einem weitestgehend gleichmäßigen Abstand zueinander auf der Wundauflage angebracht werden. Die Taschen können insbesondere auf einem oder mehreren konzentrischen, den Mittelpunkt der Wundauflage umlaufenden Kreisen angeordnet vorliegen, wobei die Taschen vorzugsweise auf jedem Kreis mit einem zueinander gleichmäßigen Abstand verteilt vorliegen. Vorzugsweise liegen die Taschen auf mindestens 30 % des Umfangs der vorgenannten konzentrisch Kreis vor, insbesondere auf mindestens 50 % des Umfangs.

Hinsicht einer Zuschneidbarkeit der Wundauflage durch den Anwender ist es dabei besonders vorteilhaft, wenn die Taschen auf der Wundauflage in mehreren, konzentrischen, den Mittelpunkt der Wundauflage umlaufenden Kreisen angeordnet vorliegen. Wenn bei einer derartigen Anordnung die weiter außen angebrachten Taschen bei Zuschneiden abgeschnitten oder zerstört werden, so können bei der Applikation der Wundauflage die weiter innen vorhandenen Taschen weiter verwendet werden.

Eine bevorzugte Ausführungsform der erfindungsgemäßen Wundauflage umfasst mindestens eine Tasche, wobei die Tasche durch das Aufbringen von flächigen Materialabschnitten, insbesondere Folienstücken, auf die im Gebrauch von der Wunde abgewandte Seite der Wundauflage gebildet wird. Die Tasche kann insbesondere durch Verkleben, thermisches Verschweißen, Verpressen oder Ultraschallschweißen auf der Wundauflage befestigt werden. Die Befestigung an der Wundauflage erfolgt am Rand des Materialabschnittes, so dass eine Außennaht hergestellt wird. Der vorwiegend zur Mitte der Wundauflage hin orientierte Rand des Materialabschnittes wird dabei nicht mit der Wundauflage verbunden, so dass eine Öffnung zum Einschieben eines chirurgischen Instrumentes oder eines Fingers vorhanden ist. Eine derartige Tasche ähnelt in ihrem Aufbau einer Hemdtasche, also einer nach drei Seiten mit einer Naht verschlossenen Tasche, welche eine Öffnung aufweist, in die ein Gegenstand eingeführt werden kann. Die im Gebrauch zur Wunde hin zeigende innere Oberfläche der Tasche wird dabei durch den wundabseitig angeordneten Folienanteil der Wundauflage gebildet, während die im Gebrauch von der Wunde weg zeigende innere Oberfläche der Tasche durch den aufgebrachten Materialabschnitt gebildet wird. Eine derartige Tasche kann auf einfache und kostengünstige Weise hergestellt werden.

Gemäß einer alternativen und gleichfalls sehr vorteilhaften Ausgestaltung der Erfindung weist die Wundauflage wundabseits eine oder mehrere Taschen auf, welche aus einem Materialabschnitt in der Form eines Kreisrings gefertigt sind. Eine solchermaßen ausgeführte Tasche eignet sich insbesondere zum Anbringen auf eine kreisförmige Abdominalwundauflage. Der die Form eines Kreisrings aufweisende Materialabschnitt wird an seinem äußeren Umfang an der Wundauflage befestigt, so dass eine kreisförmige Naht gebildet wird. Durch jeden Materialabschnitt wird jeweils eine einzige, die Mitte der Wundauflage umlaufende Tasche mit einer zur Mitte der Wundauflage hin zeigenden Öffnung gebildet. Der äußere Umfang des Kreisrings darf dabei höchstens die Größe der Wundauflage aufweisen. Der innere Umfang des Kreisrings sollte vorzugsweise so gewählt werden, dass eine Tasche mit einer Tiefe von höchstens 15 cm, insbesondere höchstens 10 cm gebildet wird. Es können mehrere konzentrisch angeordnete derartige Taschen auf die Wundauflage aufgebracht werden. Dies erleichtert die Applikation einer durch Zuschneiden an die Wundgröße angepassten Wundauflage: Nach einer Zerstörung einer weiter außen angebrachten Tasche durch das Zuschneiden kann bei der Applikation der Wundauflage eine weiter innen vorhandene Tasche verwendet werden.

Die vorgenannte Ausführungsform ist dabei nicht auf exakt kreisförmig ausgebildete Wundauflagen beschränkt, sondern kann bei geeigneter Anpassung des Materialabschnittes ebenso bei oval gestalteten Abdominalwundauflagen ausgeführt werden.

Eine weitere bevorzugte Ausführungsform der erfindungsgemäßen Wundauflage umfasst mindestens eine sackartig bzw. tütenartig ausgebildete Tasche, welche zum Aufbringen auf die im Gebrauch von der Wunde abgewandte Seite der Wundauflage vorgesehen ist. Die Tasche sollte dabei so auf die Wundauflage aufgebracht werden, dass deren Öffnung vorwiegend zur Mitte der Wundauflage hin orientiert vorliegt. Die Tasche ist vorzugsweise aus einem Folienmaterial gefertigt und sollte weiterhin vorzugsweise eine Tiefe a von höchstens 15 cm, insbesondere höchstens 10 cm aufweisen. Die Tasche kann insbesondere durch Verkleben, thermisches Verschweißen, Verpressen oder Ultraschallschweißen auf der Wundauflage befestigt werden. Die im Gebrauch zur Wunde hin zeigende innere Oberfläche der Tasche wird bei dieser Ausführungsform durch einen ersten Materialabschnitt der Tüte gebildet, während die im Gebrauch von der Wunde weg zeigende innere Oberfläche der Tasche durch einen zweiten Materialabschnitt der Tüte gebildet wird.

Gemäß einer weiteren bevorzugten Ausführungsform umfasst die erfindungsgemäße Wundauflage mindestens eine Tasche, wobei die Tasche durch Rückfaltung eines Folienanteils der Wundauflage gebildet wird. Die Tasche wird dabei insbesondere durch Rückfaltung der ersten Folie und/oder der zweiten flexiblen Folie gebildet.

Zur Gewährleistung einer ausreichenden Drainagekapazität der Wundauflage kann optional vorgesehen sein, dass die Tasche aus einem Material, insbesondere aus einem Folienmaterial gefertigt wird, welches eine Vielzahl von über die Fläche verteilten Öffnungen aufweist. Vorzugsweise sollten die in dem zur Bildung einer Tasche vorgesehenen Materialabschnitt vorhandenen Öffnungen mindestens 0,1 % und höchstens 25 % der Flächenerstreckung, vorzugsweise mindestens 10 % und höchstens 22 % der Flächenerstreckung des Materialabschnitts aufweisen.

Zur Applikation einer erfindungsgemäßen Wundauflage, kann der Anwender ein flaches chirurgisches Instrument, beispielsweise einen Bauch- und Darmspatel, in die mindestens eine Tasche einführen und sodann die durch die Tasche vorübergehend am Spatel gehaltene Wundauflage vorsichtig unter die Bauchdecke einschieben. Alternativ wäre es auch möglich, zum Applizieren der Wundauflage anstelle eines Instrumentes einen oder mehrere Finger in die mindestens eine Tasche einzuführen.

In der Praxis erweist sich weiterhin als sehr vorteilhaft erwiesen, wenn die Wundauflage auf der von der Wunde weg weisenden Seite eine oder mehrere flüssigkeitsdurchlässige Schichten zum Aufbringen auf die im Gebrauch von der Wunde abgewandte Seite der Wundauflage umfasst. Gemäß einer weiteren Ausführungsform der Erfindung umfasst die Wundauflage deshalb eine oder mehrere flüssigkeitsdurchlässige Schichten zum Aufbringen auf die im Gebrauch von der Wunde abgewandte Seite des Verbundes aus erster Folie und Leitungsmittelabschnitten oder des Verbundes aus erster Folie, Leitungsmittelabschnitten und zweiter Folie. Die flüssigkeitsdurchlässige Schicht umfasst vorzugsweise einen porösen Schaumstoff, insbesondere einen porösen Polymerschaumstoff. Besonders geeignet ist dabei ein offenzelliger Polymerschaumstoff. Im Rahmen dieser Anmeldung bedeutet der Begriff offenzellig, dass im Schaumstoff (c) mindestens 60 % offene Zellen, bevorzugt mindestens 90 % offene Zellen, mehr bevorzugt mindestens 98 % offene Zellen, insbesondere im Wesentlichen 100 % offene Zellen, bezogen auf die Gesamtzahl an Zellen, vorhanden sind.

Geeignete Materialien für einen porösen Schaumstoff umfassen beispielsweise Polyurethan, Polyurethan-Polyharnstoff-Copolymere, Polyvinylalkohol (PVA) oder Silikon.

Alternativ oder zusätzlich kann die flüssigkeitsdurchlässige Schicht textile Materialien wie Woven oder Non-woven umfassen, beispielsweise einen Vliesstoff aus synthetischen Polymeren wie Polyamid, Polyester oder Polypropylen.

Im Zusammenhang mit der vorliegenden Erfindung können die in der deutschen Patentanmeldung DE102010034819 beschriebenen porösen Schaumstoffe in besonders vorteilhafter Weise zur Herstellung der einen oder mehreren flüssigkeitsdurchlässigen Schichten eingesetzt werden. Auf den Inhalt der deutschen Patentanmeldung DE102010034819 wird hiermit Bezug genommen. Die in der DE102010034819 beschriebenen Schaumstoffe setzen beim zur Anpassung an die Wundform gegebenenfalls nötigen Zuschneiden keine oder nur im geringen Maße Schaumstoffpartikel frei. Freigesetzte Schaumstoffpartikel, welche in die Wunde gelangen, können die Wunde reizen und die Wundheilung beeinträchtigen.

In besonders vorteilhafter Weise kann der vom Anmelder Paul Hartmann AG (Heidenheim, Deutschland) vertriebene offenzellige Polyurethan-Schaumstoff VivanoMed^{®} Foam als flüssigkeitsdurchlässige Schicht eingesetzt werden.

Die mindestens eine weitere flüssigkeitsdurchlässige Schicht kann die Weichheit und Verträglichkeit der Wundauflage verbessern und einen zusätzlichen Beitrag zur Ableitung von Wundexsudat leisten.

Weiterhin ist es zur Förderung des primären Wundverschlusses insbesondere sehr vorteilhaft einen porösen Polymerschaumstoff derart anzubringen, dass der Schaumstoff in direktem Kontakt mit den Wundrändern steht.

Die mindestens eine weitere flüssigkeitsdurchlässige Schicht weist eine Dicke von 2 mm bis 50 mm, vorzugsweise von 3 mm bis 30 mm auf.

Die weitere flüssigkeitsdurchlässige Schicht, welche vorzugsweise einen porösen Polymerschaumstoff umfasst, kann dabei die gesamte Fläche des Verbundes aus Leitungsmittelabschnitten und erster Folie (oder falls vorhanden des Verbundes aus erster Folie, Leitungsmittelabschnitten und zweiter Folie) überfangen. Vorzugsweise liegt die flüssigkeitsdurchlässige Schicht nur auf einem mittigen Anteil der Fläche von erster oder - falls vorhanden - zweiter Folie vor. Insbesondere bei der Verwendung der Wundauflage als temporärer Wundverschluss hat es sich als günstig erwiesen, wenn ein poröser Polymerschaumstoff derart aufgebracht wird, dass die Ränder des Schaumstoffs in direktem Kontakt mit den Wundrändern stehen.

Falls die Leitungsmittelabschnitte durch ein zentral angeordnetes Verbindungselement verbunden sind, kann es vorteilhaft sein, wenn die weitere flüssigkeitsdurchlässige Schicht eine entsprechende Aussparung zur Aufnahme des Verbindungselementes aufweist. Die Aussparung kann dabei lediglich als Vertiefung oder alternativ als durchgehende Öffnung ausgebildet sein.

Im Zusammenhang mit der vorliegenden Erfindung kann mehr als eine flüssigkeitsdurchlässige Schicht auf der im Gebrauch von der Wunde abgewandten Seite des Verbundes aus erster Folie und Leitungsmittelabschnitten oder des Verbundes aus erster Folie, Leitungsmittelabschnitten und zweiter Folie vorgesehen sein, insbesondere mehr als eine Schicht eines porösen Polymerschaumstoffs. Die mehreren Schichten können dabei unterschiedliche Abmessungen aufweisen und in unterschiedlichen Dicken vorliegen. Für die Unterdruckbehandlung einer typischen Abdominalwunde hat es sich als vorteilhaft erwiesen, wenn zwei Schichten eines offenzelligen Polyurethanschaum mit einer Schichtdicke von jeweils 16 mm appliziert werden.

Die erfindungsgemäße Wundauflage kann weiterhin ein luftundurchlässiges Abdeckmaterial zum luftdichten Verschließen der Wunde und der Wundumgebung umfassen, wobei das Abdeckmaterial vorzugsweise einen Kleberand zum Befestigen des Abdeckmaterials auf der die Wunde umgebenden intakten Haut, aufweist. Unter "luftdichten Verschließen" soll hier nicht verstanden werden, dass keinerlei Gasaustausch zwischen dem Wundraum und seiner Umgebung stattfindet. Vielmehr ist mit "luftdichten Verschließen" in diesem Zusammenhang gemeint, dass unter Berücksichtigung der eingesetzten Unterdruckquelle der für die Unterdrucktherapie von Wunden nötige Unterdruck aufrechterhalten werden kann. Es können deshalb auch Abdeckmaterialien eingesetzt werden, welche eine geringfügige Gas-Permeabilität aufweisen, so lange der für die Unterdrucktherapie notwendige Unterdruck aufrechterhalten werden kann.

Das Abdeckmaterial wird in der Wundumgebung oder am Wundrand so befestigt, dass ein luftdichter Wundverschluss gewährleistet ist. Dabei kann es zweckmäßig sein, wenn das Abdeckmaterial vollflächig selbstklebend ausgestattet ist oder einen selbstklebenden Rand aufweist. Alternativ können Befestigung und Abdichtung beispielsweise mit einer Klebefolie, mit einem flüssigen Kleber oder mit einer Abdichtmasse erfolgen.

In einer bevorzugten Ausführungsform der Erfindung umfasst das Abdeckmaterial zum luftdichten Verschließen der Wunde ein wasserunlösliches Polymer, oder eine Metallfolie.

In einer besonders bevorzugten Ausführungsform der Erfindung handelt es sich bei dem wasserunlöslichen Polymer um Polyurethan, Polyester, Polypropylen, Polyethylen, Polyamid oder Polyvinylchlorid, Polyorganosiloxan (Silikon), oder um eine Mischung daraus.

Als Abdeckmaterial können auch Fertigprodukte eingesetzt werden, welche die vorstehend genannten Eigenschaften aufweisen.

Als besonders geeignetes Abdeckmaterial für die erfindungsgemäße Vorrichtung hat sich Polyurethanfilm der Marke Hydrofilm^{®} (Paul Hartmann AG, Deutschland) oder Visulin ^{®} (Paul Hartmann AG, Deutschland) erwiesen.

Im Zusammenhang mit der erfindungsgemäßen Wundauflage wird des weiteren ein Unterdruck-Anschlussstück zur funktionellen Verbindung des Wundraums mit einer außerhalb der Wundauflage befindlichen Unterdruckquelle beansprucht, wobei das Unterdruck-Anschlussstück derart gestaltet ist, dass ein Unterdruck im Wundraum herstellbar ist und Flüssigkeiten aus dem Wundraum absaugbar sind. Das Unterdruck-Anschlussstück wird bei der Verwendung der Wundauflage in der Unterdrucktherapie von Wunden vorzugsweise auf die von der Wunde abgewandte Seite des luftundurchlässigen Abdeckmaterials angebracht, wobei das Abdeckmaterial geeignete Öffnungen aufweist. Unterdruck-Anschlussstücke sind dem Fachmann auch unter der Bezeichnung "Port" bekannt. Das Unterdruck-Anschlussstück umfasst üblicherweise eine Verbindungsleitung, und einen Unterdruckadapter, um mit den weiteren Komponenten des Unterdrucksystems verbindbar zu sein.

Gemäß einer weiteren bevorzugten Ausführungsform werden Abdeckmaterial und das Mittel zur funktionellen Verbindung des Wundraums mit einer außerhalb des Abdeckmaterials befindlichen Unterdruckquelle bereits gebrauchsfertig miteinander verbunden zur Verfügung gestellt. Es ist ganz besonders bevorzugt, dass diese Ausführungsform eine Folie aus einem oder mehreren wasserunlöslichen Polymeren enthält, welche einen selbstklebenden Rand aufweist, da diese Anordnung das Anlegen des Verbandes wesentlich erleichtert.

Für die in der vorliegenden Erfindung beschriebene Wundauflage sind die in den Patentanmeldungen WO2011091947, WO2011091952 und WO2011076340, sowie die in der deutschen Patentanmeldung DE102011108726.9 (zum Zeitpunkt der vorliegenden Anmeldung noch nicht veröffentlicht) offenbarten Unterdruck-Anschlussstücke besonders geeignet.

Gemäß einer alternativen Ausführungsform kann die funktionelle Verbindung des Wundraums mit einer außerhalb des Abdeckmaterials befindlichen Unterdruckquelle mit mindestens einer Verbindungsleitung hergestellt werden. Die mindestens eine Verbindungsleitung kann durch das Abdeckmaterial hindurchgeführt werden oder unter dem Rand des Abdeckmaterials durchgeführt werden. In beiden Fällen ist die Durchtrittsstelle luftdicht abzudichten, so dass der gewünschte Unterdruck im Verband aufrechterhalten werden kann. Als Abdichtmittel eignet sich beispielsweise eine Klebefolie, eine Klebemasse, oder ein Klebestreifen.

Bei der Verbindungsleitung kann es sich beispielsweise um einen Schlauch, um ein Rohr oder um einen anderen Körper mit einem Hohlraum handeln. Ein geeigneter Schlauch ist beispielsweise ein Silikon-Drainageschlauch.

Die erfindungsgemäße Wundauflage kann weiterhin ein Mittel umfassen, so dass der in der Vorrichtung tatsächlich vorhandene Unterdruck überprüfbar und gegebenenfalls einstellbar ist. Das Mittel kann sich im Wundraum oder an einer anderen geeigneten Stelle befinden. Alternativ ist es auch möglich einen Drucksensor in der Unterdruckleitung zwischen Wundverband und der Unterdruckquelle anzubringen.

Es ist vorgesehen, dass die vorgenannten Komponenten den die Wundbehandlung durchführenden Ärzten und Fachkräften als gebrauchsfertiges Set ("Kit") zur Verfügung gestellt werden. Die Erfindung bezieht sich daher auch auf ein gebrauchsfertiges Set zur Unterdruck-Wundbehandlung umfassend
a) eine Wundauflage nach einem oder mehreren der Ansprüche 1 bis 17,
b) mindestens eine flüssigkeitsdurchlässige Schicht zum Aufbringen auf die im Gebrauch von der Wunde abgewandte Seite der Wundauflage, wobei die mindestens eine flüssigkeitsdurchlässige Schicht bevorzugt eine oder mehrere flächenförmige Polster aus einem porösen Polymerschaumstoff, insbesondere aus PU, PVA oder Silikon umfasst,
c) ein luftundurchlässiges Abdeckmaterial zum luftdichten Verschließen der Wunde und der Wundumgebung, wobei das Abdeckmaterial vorzugsweise einen Kleberand aufweist,
d) ein Unterdruck-Anschlussmittel zur funktionellen Verbindung des Wundraums mit einer außerhalb des Abdeckmaterials befindlichen Unterdruckquelle, so dass ein Unterdruck im Wundraum herstellbar ist und Flüssigkeiten aus dem Wundraum absaugbar sind, wobei das Unterdruck-Anschlussmittel vorzugsweise zum Anbringen auf die im Gebrauch von der Wunde abgewandte Außenseite des Abdeckmaterials vorgesehen ist, und wobei die Komponenten a) bis d) steril verpackt vorliegen können

Für das gebrauchsfertige Set besonders geeignete Unterdruck-Anschlussstücke sind in den vorgenannten Patentanmeldungen Anmeldenummer WO2011091947, WO2011091952, WO2011076340 und DE102011108726.9 (zum Zeitpunkt der vorliegenden Anmeldung noch nicht veröffentlicht) beschrieben.

Weiterhin kann das Set optionale Bestandteile wie beispielsweise eine oder mehrere zusätzliche flächenförmige Elemente einer flüssigkeitsdurchlässigen Schicht, Klebemittel zum Fixieren des Verbands, Abdichtmittel zur Herstellung einer luftundurchlässigen Abdichtung des Verbandes, Drucksensoren, Verbindungselemente für Drucksensoren, Schläuche, Verbindungsstücke für Schläuche, Desinfektionsmittel, Hautpflegemittel, pharmazeutische Zubereitungen oder eine Gebrauchsanweisung enthalten.

Vorzugsweise umfasst das Set weiterhin eine gebrauchsfertige Unterdruckeinheit. Die Unterdruckeinheit kann Komponenten wie beispielsweise eine Pumpe, ein oder mehrere Flüssigkeitsbehälter, eine Steuereinheit, eine Stromversorgung, elektrische Anschlussmitteln und Schläuche enthalten. Die Unterdruckeinheit kann auch eine Vorrichtung zur funktionellen Verbindung des Unterdruckverbandes mit einer vorhandenen stationären Unterdruckquelle enthalten.

Für das gebrauchsfertige Set besonders geeignete Unterdruck-Einheiten sind in den Patentanmeldungen WO2011018133 und WO2011018132 beschrieben. Ein für das Set besonders geeignete Unterdruck-Einheit ist unter der Bezeichnung VivanoTec^{®} (Hersteller Paul Hartmann AG, Heidenheim, Deutschland) kommerziell erhältlich.

Vorzugsweise werden alle Komponenten, bei denen dies aus medizinischer Sicht notwendig ist, steril abgepackt zur Verfügung gestellt. Der Vorteil des gebrauchsfertigen Sets besteht darin, dass der Unterdruckverband schnell, standardisiert und unkompliziert angelegt werden kann. Ein weiterer Vorteil besteht darin, dass alle im Wundbereich eingesetzten Bestandteile des Sets bereits sterilisiert zur Verfügung gestellt werden können.

### Figurenlegende

- 1.: Erste flexible Folie mit einer Vielzahl von über die Fläche verteilten Öffnungen
- 2.: Leitungsmittelabschnitt
- 3.: Hohlraum (Lumen) in Leitungsmittelabschnitt
- 4.: Zweite flexible Folie
- 5.: Endständige Öffnung in Leitungsmittelabschnitt
- 6.: Öffnung in erster flexibler Folie
- 7.: Haft- bzw. Befestigungspunkt zwischen erster und zweiter Folie
- 8.: Verbindungselement
- 10.: Wundauflage zur Verwendung bei der Unterdrucktherapie von Wunden, insbesondere zur Behandlung abdominaler Wunden
- 11.: Flüssigkeitsdurchlässige Schicht
- 12.: Zwischenraum zwischen Wundgrund (beispielsweise freigelegte innere Organe oder das Omentum majus) und Bauchdecke
- 13.: Wundgrund, insbesondere freigelegte innere Organe oder das Omentum majus
- 14.: Bauchdecke
- 15.: Wundrand
- 16.: Luftundurchlässiger Abdeckfilm
- 17.: Öffnung in Abdeckfilm
- 18.: Unterdruck-Anschlussmittel (Port)
- 19.: Unterdruckleitung
- 20.: Kanister für Wundexsudat
- 21.: Unterdruckquelle
- 22.: Adhäsive Schicht, welche die erste Folie mit einem Leitungsmittelabschnitt verbindet
- 25.: Zur Mitte der Wundauflage hin offene Tasche
- 26.: Naht bzw. Verklebungsbereich von Tasche mit erster oder zweiter flexibler Folie
- 27.: Öffnung der Tasche
- 28.: Lumen der Unterdruckleitung
- 40.: Vorrichtung zur Verwendung bei der Unterdrucktherapie von Wunden, insbesondere zur Behandlung abdominaler Wunden
- 50.: Messvorrichtung zur Testung der Drainagekapazität einer Wundauflage
- 51.: Erster Hohlkörper
- 52.: Zweiter Hohlkörper
- 53.: Erste Öffnung im zweiten Hohlkörper
- 54.: Zweite Öffnung im zweiten Hohlkörper
- 521.: Zusätzliche Lage eines flexiblen Materials
- 531.: Fluidquelle
- 532.: Leitungsmittel für Zufuhr der Blutersatzlösung
- 533.: Unterdruckquelle
- 534.: Sammelkanister
- 535.: Drainageleitung
- 536.: Unterdruckanschlussstück (Port)
- 537.: Erste flexible Folie
- 538.: Schaumstofflage
- 539.: Selbstklebende Abdeckfolie
- 540.: Messvorrichtung für Fluidvolumen

### Figuren

Nachstehend wird die erfindungsgemäße Wundauflage bzw. Vorrichtung zur Unterdrucktherapie von Wunden anhand schematischer Zeichnungen (nicht maßstabsgetreu) näher erläutert. Die Erfindung soll jedoch nicht auf die in den Zeichnungen oder in der Beschreibung der Zeichnung dargestellten Ausgestaltungen reduziert verstanden werden. Vielmehr umfasst die erfindungsgemäße Vorrichtung auch Kombinationen der Einzelmerkmale der alternativen Formen.
Fig. 1 a zeigt eine Ausführungsform der erfindungsgemäßen Wundauflage zur Verwendung bei der Unterdrucktherapie in der Aufsicht auf die wundabgewandte Seite der Wundauflage.
Fig. 1b zeigt eine weitere Ausführungsform der erfindungsgemäßen Wundauflage zur Verwendung bei der Unterdrucktherapie in der Aufsicht auf die wundabgewandte Seite der Wundauflage.
Fig. 1c zeigt eine weitere Ausführungsform der erfindungsgemäßen Wundauflage zur Verwendung bei der Unterdrucktherapie in der Aufsicht auf die wundabgewandte Seite der Wundauflage.
Fig. 2 zeigt einen Querschnitt der in Fig. 1 a dargestellten Wundauflage entlang der Linie A-A.
Fig. 3 zeigt einen Querschnitt einer weiteren Ausführungsform der erfindungsgemäßen Wundauflage. Die Wundauflage umfasst eine erste flexible Folie und zusätzlich eine zweite flexible Folie. Der Schnitt wurde durch einen peripheren Bereich der Wundauflage geführt, entsprechend der Linie A-A von Fig. 1 a.
Fig. 4 zeigt eine bevorzugte Ausführungsform einer an eine abdominale Wunde angelegten Vorrichtung zur Verwendung bei der Unterdrucktherapie von Wunden im Querschnitt. Der Schnitt wurde durch die Mitte der Wundauflage geführt, entsprechend der Linie B-B von Fig. 1 b.
Fig. 5 zeigt eine Messvorrichtung für Wundverbände ("Wundsimulator") einschließlich eines zu testenden NPWT-Abdominalverbandes im Querschnitt.
Fig. 6 zeigt eine Ausführungsform eines Leitungsmittelabschnittes im Querschnitt.

### Ausführliche Beschreibung der Erfindung

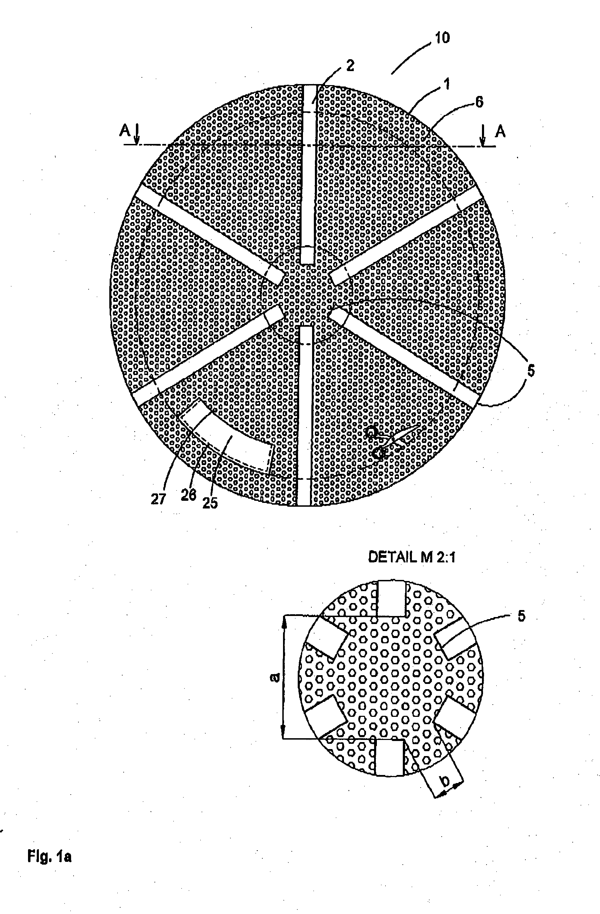

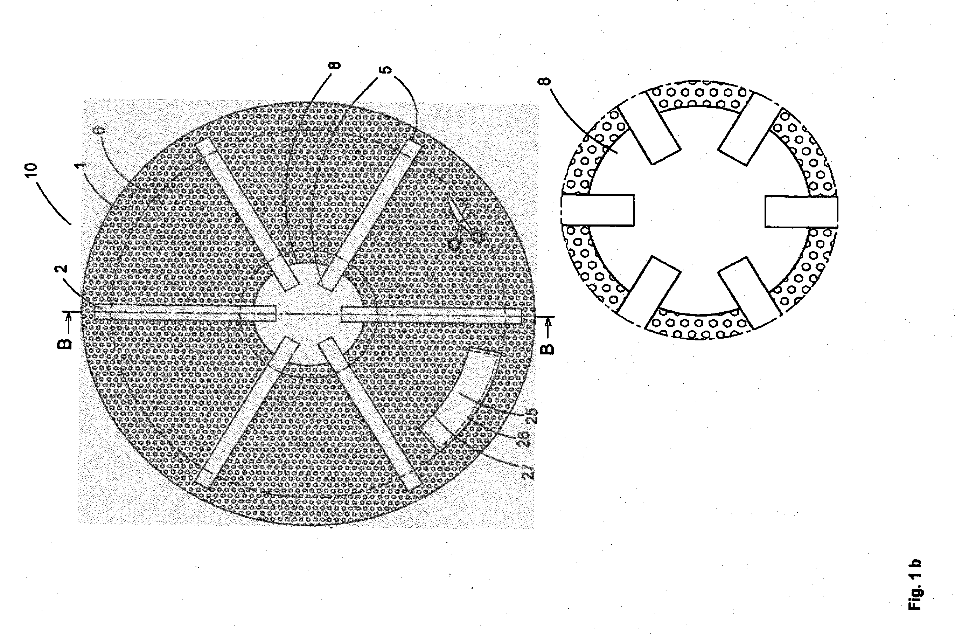

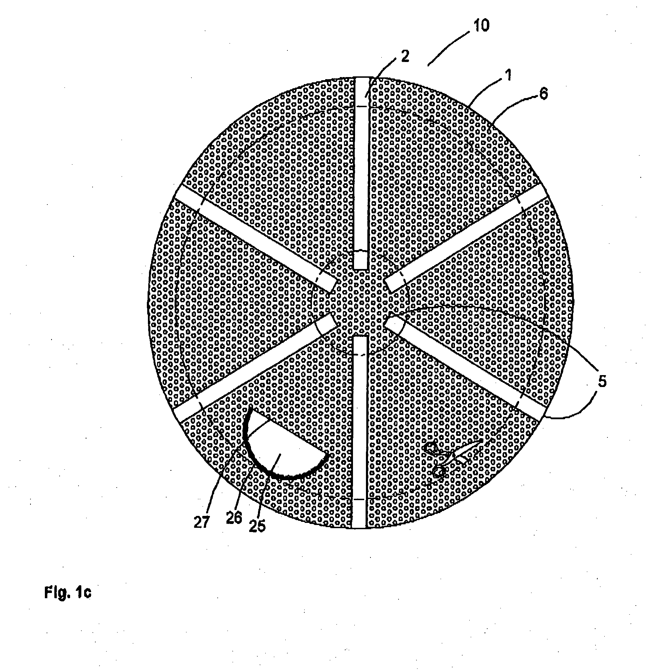

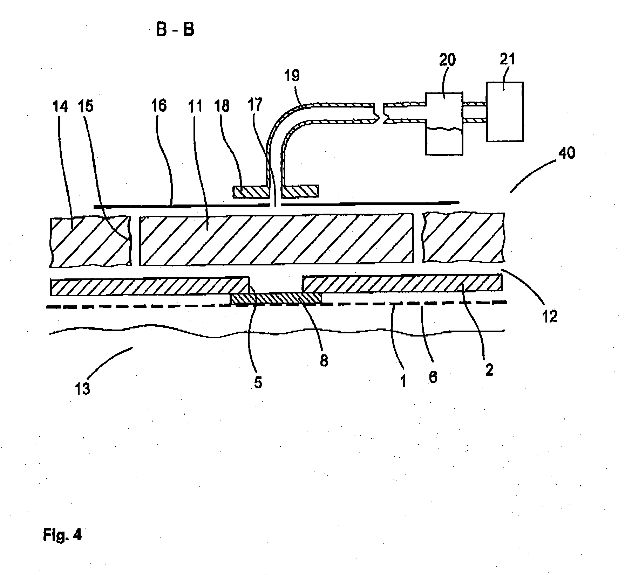

Die in Fig. 1 a und Fig. 1b dargestellten Ausführungsformen der Wundauflage umfassen jeweils eine erste flexible Folie (1), auf welche Leitungsmittelabschnitte (2) aufgebracht sind. Die erste flexible Folie (1) weist eine erste und einer zweite Seite auf, wobei die erste Seite zum Aufbringen auf den Wundgrund (siehe Fig. 4, Bezugszeichen 13), insbesondere auf freigelegte innere Organe oder auf das Omentum majus, vorgesehen ist und somit als Wundkontaktschicht dienen kann. Die erste Folie (1) weist weiterhin eine Vielzahl von über die Fläche verteilten Öffnungen (6) auf. Bei der in Fig. 1a und Fig. 1b dargestellten Ausführungsform der Erfindung sind kreisförmige Öffnungen (Löcher) vorgesehen, wobei die Summe der offenen Fläche der in der ersten Folie (1) vorhandenen Öffnungen (6) etwa 14 % Flächenerstreckung der Folie beträgt. Die Summe der offenen Fläche der in der Folie (1) vorhandenen Öffnungen (6) sollte jedoch mindestens 0,1 % und höchstens 25 % der Flächenerstreckung, vorzugsweise mindestens 10 % und höchstens 18 % der Flächenerstreckung, insbesondere mindestens 13 % und höchstens 15 % der Flächenerstreckung der Folie betragen. Derartige Öffnungen (6) können beispielsweise durch Stanzen erzeugt werden. Auf der ersten flexiblen Folie (1) sind bei den in Fig. 1 a und Fig. 1b schematisch dargestellten Ausführungsformen jeweils sechs Leitungsmittelabschnitte (2) aus einem biegsamen elastomeren Material mit einer Dickenerstreckung (H) von ca. 6 mm sternförmig auf einer ersten flexiblen Folie (1) angeordnet. Die Breite (B) der Leitungsmittelabschnitte (2) beträgt ca. 20 mm. Die Leitungsmittelabschnitte (2) weisen mit Ausnahme der endständigen Öffnungen (5) keine weiteren Öffnungen auf. Die Leitungsmittelabschnitte (2) sind mit der ersten Folie (1) unlösbar verbunden, beispielsweise durch Verkleben. Der Leitungsmittelabschnitt (2) ist dabei flach ausgebildet und mit mindestens 10 % seiner senkrecht auf die erste Folie (1) projizierten Fläche mit derselben unlösbar und flächenhaft verbunden. Der Leitungsmittelabschnitt (2) ist aus einem elastomeren Material gefertigt, welches eine Shore A Härte von 60 aufweist. Geeignete Materialien umfassen EVA, PU, PVC, PE, PET, PTFE, TPE, Silikon oder eine Mischung daraus. Die Shore A Härte soll in jedem Falle mindestens 20 und höchstens 70 betragen, vorzugsweise mindestens 30 und höchstens 65, insbesondere soll ein Leitungsmittelabschnitt (2) eine Shore A Härte von mindestens 40 und höchstens 62 aufweisen. Ein Leitungsmittelabschnitt (2) kann durch Ablängen eines längeren Drainageschlauches, welcher für medizinische Zwecke vorgesehen ist, gewonnen werden.

Wie durch das Scherensymbol in Fig. 1 a und Fig. 1b angedeutet, kann die Wundauflage durch Zuschneiden auf die für die Behandlung der Wunde benötigte Größe angepasst werden, wobei sowohl die erste Folie (1) als auch ggf. ein oder mehrere Leitungsmittelabschnitte (2) durchtrennt werden können.

Bei der in Fig. 1 a schematisch dargestellten Ausführungsform der Erfindung sind die eine endständige Öffnung (5) aufweisenden Endstücke der sternförmig auf der ersten Folie (1) angeordneten Leitungsmittelabschnitte (2) voneinander beabstandet. Der in Fig. 1 a mit dem Bezugszeichen b gekennzeichnete Abstand zu den benachbarten Leitungsmittelabschnitten (2) beträgt in der beispielhaft gezeigten Ausführungsform jeweils ca. 2 cm, kann jedoch auch geringer oder größer sein. Als günstig hat sich ein Abstand b von 0,5 bis 7 cm erwiesen. Der Abstand a der zur Mitte der Wundauflage hin zeigenden Enden der auf der ersten Folie (1) gegenüber angeordneten Leitungsmittelabschnitte (2) ergibt sich aus der Anzahl der vorhandenen Leitungsmittelabschnitte und deren Anordnung. Bei der hier beispielhaft vorgestellten Ausführungsform beträgt er ca. 8 cm.

Es ist jedoch auch möglich, dass die kreuz- oder sternförmig auf der ersten Folie (1) angeordneten Leitungsmittelabschnitte (2) im Zentrum der ersten Folie (1) aneinander stoßen (nicht dargestellt).

Die nach außen und damit zur Peripherie der Wundauflage hin orientierten und gleichfalls eine endständige Öffnung (5) aufweisenden Enden der sechs Leitungsmittelabschnitte (2) erstrecken sich bei der in Fig. 1 a dargestellten Ausführungsform bis zum Rand der ersten Folie (1). Die nach außen hin orientierten Enden (5) der Leitungsmittelabschnitte (2) können jedoch auch gegenüber dem Rand der ersten Folie (1) zurückgesetzt sein, wie in Fig. 1 b gezeigt. Dabei kann beispielsweise ein Abstand vom Ende eines Leitungsmittelabschnittes (2) bis zum Rand der ersten Folie (1) von 1 bis 10 cm vorhanden sein.

In Fig. 1 a ist eine Tasche (25) dargestellt, welche das gleichmäßige Aufbringen und Auslegen des Verbandes auf freigelegte innere Organe oder auf das Omentum majus erleichtern kann. Eine derartige Tasche (25) kann durch das Aufbringen von zusätzlichen Materialstücken, insbesondere Folienstücken, auf die erste Folie (1) gebildet werden. Eine Tasche (25) könnte auch durch Einfalten der ersten Folie (1) gebildet werden. Die Tasche (25) ist an ihren Rändern mit einer Naht bzw. einem Verklebungsbereich (26) an der ersten Folie befestigt, wobei jedoch die zur Mitte der Wundauflage hin zeigende Seite der Tasche eine Öffnung aufweist (27). In die zur Mitte der Wundauflage hin zeigende Öffnung kann der behandelnde Arzt einen Bauch- oder Darmspatel oder gegebenenfalls einen Finger einführen und sodann den Randbereich der Wundauflage vorsichtig zwischen Bauchdecke und den Wundgrund einbringen. Die in Fig. 1 a gezeigte Tasche (25) weist die Form eines Kreisringsektors auf, wobei alternative Ausführungen der Tasche (25) mit einer gleichfalls zur Mitte der Wundauflage hin zeigenden Öffnung denkbar sind, beispielsweise die in Fig. 1 c dargestellte Form. In Fig. 1 a ist nur eine Tasche (25) beispielhaft dargestellt. In der Praxis ist eine Vielzahl derartiger Taschen (25), vorzugsweise auf mindestens 30 % des Umfangs der Wundauflage vorgesehen. Bei dem in Fig. 1 a gezeigten Beispiel wäre es vorteilhaft, in jedem des zwischen den sechs Leitungsmittelabschnitten (2) gebildeten Segments eine Tasche (25) aufzubringen, so dass insgesamt sechs Taschen (25) vorhanden sind (nicht dargestellt). Es wäre auch möglich ein oder mehrere zusätzliche, in konzentrisches Kreisen angeordnete Reihen von Taschen (25) anzubringen (in Fig. 1 a nicht dargestellt), so dass auch nach dem Zuschneiden (insofern dabei Taschen teilweise oder vollständig abgeschnitten werden) der Wundauflage weiter innen angeordnete Taschen vorhanden sind. Die Tiefe einer zum Erleichtern des Auflegens der Wundauflage vorgesehenen Tasche (25) beträgt zweckmäßigerweise mindestens 0,5 cm und höchstens 15 cm.

Die in Fig 1b gezeigte Ausführungsform umfasst neben den in Fig 1 a vorhandenen Komponenten ein plattenförmiges Verbindungselement (8) mit einem Durchmesser von 5 cm, welches auf die zweite Seite der ersten flexiblen Folie (1) aufgebracht werden kann. An dem vorzugsweise aus einem elastomeren Material hergestellten Verbindungselement (8) können die eine endständige Öffnung (5) aufweisenden Endbereiche der Leitungsmittelabschnitte (2) unlösbar befestigt werden. Die Leitungsmittelabschnitte (2) werden vorzugsweise auf der im Gebrauch von der Wunde weg weisende Seite des Verbindungselementes (8) befestigt. Die eine endständige Öffnung (5) aufweisenden Endstücke der sternförmig auf der ersten Folie (1) angeordneten Leitungsmittelabschnitte (2) können im Zentrum aneinander stoßen, oder wie in Fig. 1b gezeigt, voneinander beanstandet sein.

Fig. 1c zeigt eine weitere Ausführungsform der erfindungsgemäßen Wundauflage, welche eine alternative und gleichfalls vorteilhafte Ausgestaltung der Tasche (25) veranschaulicht, ansonsten jedoch die in Fig. 1 a dargestellten Strukturelemente aufweist. Die Tasche (25) der in Fig. 1c gezeigten Ausführung weist eine bogenförmige Naht auf. Die zur Mitte der Wundauflage hin zeigende Öffnung (27) ist zum Einführen eines Bauch- oder Darmspatels oder gegebenenfalls eines Fingers vorgesehen. Wie bei Fig. 1 a ist nur eine Tasche (25) beispielhaft dargestellt. In der Praxis sollte jedoch eine Vielzahl derartiger Taschen (25) vorhanden sein, vorzugsweise in jedem der sechs zwischen den Leitungsmittelabschnitten (2) gebildeten Segmenten (nicht dargestellt).

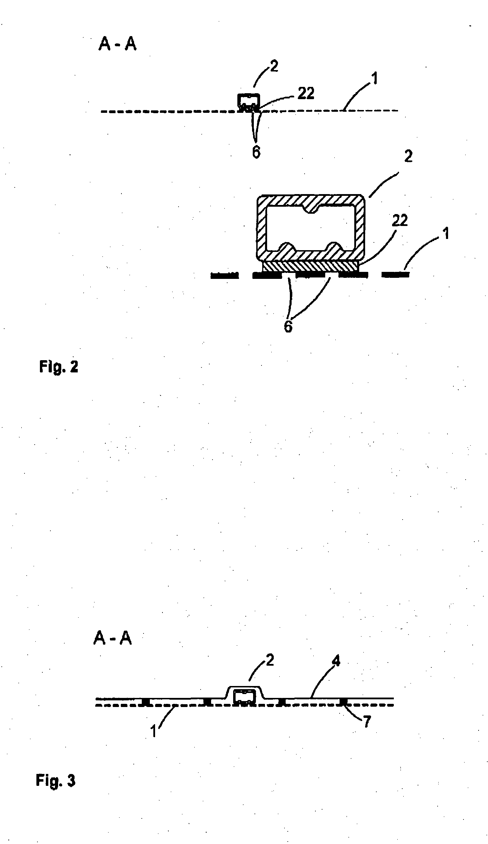

Fig. 2 zeigt einen nicht maßstabsgetreuen Querschnitt der in Fig. 1 a dargestellten Wundauflage entlang der Linie A-A. Ein Leitungsmittelabschnitt (2) mit einem im Wesentlichen rechteckigen Querschnitt ist auf einer ersten flexible Folie (1), welche eine Vielzahl von über die Fläche verteilten Öffnungen (6) aufweist, unlösbar befestigt. Der Leitungsmittelabschnitt (2) weist im beispielhaft gezeigten Falle nur einen einzigen durchgehenden Hohlraum (3) auf. Der Leitungsmittelabschnitt (2) kann auf der Innenseite Vorwölbungen oder Verdickungen aufweisen, die der Stabilisierung des Leitungsmittels (2) im Unterdruck dienen.

Zwischen der zweiten Seite der Folie (1) und dem Leitungsmittelabschnitt (2) befindet sich eine Klebeschicht (22), mittels der der Leitungsmittelabschnitt (2) mit ca. 90 % seiner senkrecht auf die erste Folie projizierten Fläche mit der zweiten Seite der ersten Folie unlösbar und flächenhaft verbunden ist. Im Rahmen der Erfindung sind jedoch auch alternative Mittel zur Befestigung des Leitungsmittelabschnittes (2) an der Folie (1) möglich.

Fig. 3 zeigt eine weitere Ausführungsform der erfindungsgemäßen Wundauflage. Die Wundauflage unterscheidet sich von der in Fig. 2 gezeigten Ausführungsform dadurch, dass zusätzlich eine zweite flexible Folie (4) vorhanden ist, wobei die zweite Folie (4) auf die im Gebrauch von der Wunde abgewandte Seite des Verbundes aus erster Folie (1) und Leitungsmittelabschnitten (2) unlösbar aufgebracht ist. Erste flexible Folie (1) und zweite flexible Folie (4) sind durch Haft- bzw. Befestigungspunkte (7) unlösbar miteinander verbunden. Der Leitungsmittelabschnitt (2) wird in einer durch die erste Folie (1) und die zweite Folie (4) gebildeten Tasche gehalten, so dass bei der in Fig. 3 gezeigten Ausführungsform auf eine adhäsive Schicht zwischen Leitungsmittelabschnitt (2) und erster Folie (1) verzichtet werden kann. Die zweite flexible Folie (4) bewirkt somit die Befestigung des Leitungsmittelabschnittes (2) auf der ersten Folie (1) dar. Eine punktförmige Verklebung von erster Folie (1) und zweiter Folie (4) erweist sich als besonders vorteilhaft, da zwischen erster Folie (1) und zweiter Folie (4) ein labyrinthartiger Hohlraum gebildet wird, welcher die Drainagekapazität der Wundauflage weiter verbessern kann.

Fig. 4 zeigt eine an eine abdominale Wunde angelegte Vorrichtung (40) zur Unterdrucktherapie von Wunden im Querschnitt. Die Vorrichtung umfasst eine wie vorstehend näher beschriebene erfindungsgemäße Wundauflage (10) mit erster Folie (1) und Leitungsmittelabschnitten (2). Die erste Seite der ersten Folie (1) wird auf den Wundgrund (13), insbesondere auf freigelegte innere Organe oder auf das Omentum majus, aufgebracht. Erste Folie (1) und darauf aufgebrachte Leitungsmittelabschnitte (2) werden üblicherweise in den zwischen Wundgrund (13) und Bauchdecke (14) gebildeten Zwischenraum eingeschoben. Gemäß einer in Fig. 4 nicht dargestellten Ausführungsform der Wundauflage kann die erste Folie (1) einen Randbereich aufweisen, auf den keine Leitungsmittelabschnitte (2) aufgebracht sind. In diesem Falle ist es möglich, dass nur die erste Folie (1), jedoch keine Leitungsmittelabschnitte (2) in den zwischen Wundgrund (13) und Bauchdecke (14) gebildeten Zwischenraum eingeschoben werden.

Das Einschieben der Wundauflage in den zwischen Wundgrund (13) und Bauchdecke (14) gebildeten Zwischenraum (12) kann erleichtert werden, wenn randständige und zur Mitte der Wundauflage hin offene Taschen (25) auf der Wundauflage vorgesehen sind (in Fig. 4 nicht dargestellt, siehe Fig. 1 a - c). Der behandelnde Arzt kann einen Bauch- oder Darmspatel oder gegebenenfalls einen Finger in die Taschen einführen und sodann den Randbereich der Wundauflage zwischen Bauchdecke (14) und den Wundgrund (13) einbringen.

Auf der im Gebrauch von der Wunde abgewandten Seite des Verbundes aus erster Folie (1) und Leitungsmittelabschnitten (2) liegt eine flüssigkeitsdurchlässige Schicht (11) vor, bei der sich insbesondere um einen porösen Polymerschaumstoff handelt. Vorzugsweise wird ein offenzelliger Polyurethan-Schaumstoff verwendet. Je nach Tiefe der Wunde können mehrere Schichten der flüssigkeitsdurchlässigen Schicht (11) vorhanden sein (nicht dargestellt). Die flüssigkeitsdurchlässige Schicht (11) wurde durch Zuschneiden an die Größe der Wunde angepasst, so dass der Rand der flüssigkeitsdurchlässige Schicht (11) in direktem Kontakt mit dem Wundrand (15) steht. Es ist bekannt, dass der Kontakt des Wundrandes (15) mit einem porösen Polymerschaum (11) das Wachstum des Wundrandgewebes fördert. Die Vorrichtung (4) umfasst weiterhin ein luftundurchlässiges Abdeckmaterial (16) zum luftdichten Verschließen der Wunde, sowie ein auf das luftundurchlässige Abdeckmaterial (16) auf der von der Wunde abgewandten Seite aufbringbares Mittel (18), insbesondere ein Unterdruck-Anschlussstück (Port), zur funktionellen Verbindung des Wundraums mit einer außerhalb des Abdeckmaterials (16) befindlichen Unterdruckquelle (21). Das Unterdruck-Anschlussstück (18) wird im Bereich einer in das luftundurchlässige Abdeckmaterial (16) eingebrachten Öffnung (17) befestigt. Bei Betrieb der Unterdruckquelle (21), welche über einen Auffangkanister für Wundexsudat (20) und einer Unterdruckleitung (19) mit dem Port (18) Fluid-leitend verbunden ist, kann Unterdruck im Wundraum hergestellt und Flüssigkeiten aus dem Wundraum abgesaugt werden. Aus dem Wundraum über die Unterdruckleitung (19) abgesaugtes Wundexsudat wird im Kanister (20) gesammelt.

Bei Anlegen eines Unterdrucks kann Wundexsudat vom Wundgrund (13) über Öffnungen (6) in der ersten Folie (1) zur flüssigkeitsdurchlässigen Schicht (11) gelangen. Zusätzlich kann Wundexsudat, insbesondere vom peripheren Bereich des Wundgrundes (13), durch einen Leitungsmittelabschnitt (2) zum zentralen Bereich der Wundauflage geleitet werden und dort in die flüssigkeitsdurchlässige Schicht (11) übertreten. Aus der flüssigkeitsdurchlässigen Schicht (11) erfolgt ein Abtransport des Fluids durch eine Öffnung (17) im Abdeckfilm (16) zum Unterdruck-Anschlussstück (18) und durch die Unterdruckleitung (19) weiter zum Kanister (20).

Im Zusammenhang mit der vorliegenden Erfindung besonders geeignete tragbare Unterdruck-Einheiten, welche Kanister (20) und Unterdruckquelle (21) trennbar in einer einzigen Einheit umfassen, sind in den bereits erwähnten Patentanmeldungen Anmeldenummer WO2011018133 und WO 2011018132 beschrieben. Hinsichtlich geeigneter Unterdruckanschlussmittel (18) wird auf die bereits erwähnten Patentanmeldungen WO2011091947, WO2011091952, WO2011076340 und DE102011108726.9 (zum Zeitpunkt der vorliegenden Anmeldung noch nicht veröffentlicht) verwiesen.

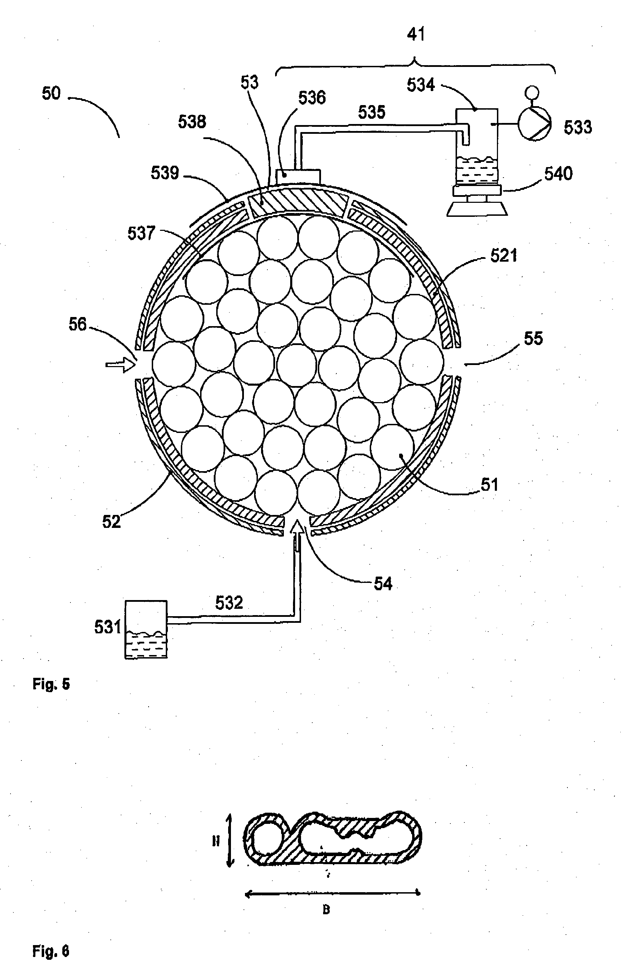

Fig. 6 zeigt beispielhaft eine Ausführungsform eines Leitungsmittelabschnittes (2) im Querschnitt mit Dickenerstreckung (H) und Breite (B). Der in Fig. 6 dargestellte Leitungsmittelabschnitt weist zwei durchgehende, nicht miteinander verbundene Hohlräume auf. Das Verhältnis der Breite B des Leitungsmittelabschnittes zu seiner Dickenerstreckung H sollte vorzugsweise mindestens 1,25 betragen. Bei dem in Fig. 6 dargestellten Beispiel beträgt das Verhältnis der Breite B des Leitungsmittelabschnittes zu seiner Dickenerstreckung H ca. 3,7. Im Zusammenhang mit der Erfindung können Leitungsmittelabschnitte mit nur einem durchgehenden Hohlraum oder aber mit mehreren durchgehenden Hohlräumen verwendet werden.

### Herstellung der Wundauflage

Die Erfindung betrifft weiterhin ein Verfahren zur Herstellung einer Wundauflage für die Unterdrucktherapie, wobei die Wundauflage insbesondere zur Behandlung abdominaler Wunden vorgesehen ist, umfassend die Schritte
a) Bereitstellen einer ersten flexible Folie (1) mit einer ersten und einer zweiten Seite, wobei die erste Seite zum Aufbringen auf den Wundgrund (13), insbesondere auf freigelegte innere Organe oder auf das Omentum majus, vorgesehen ist und wobei die Folie (1) eine Vielzahl von über die Fläche verteilten Öffnungen (6) aufweist,
b) Bereitstellen von mindestens drei Leitungsmittelabschnitten (2) aus einem biegsamen elastomeren Material mit einer Dickenerstreckung (H) von höchstens 20 mm, wobei jeder der Leitungsmittelabschnitte (2) mindestens einen durchgehenden Hohlraum (3) aufweist, und wobei jeder der Leitungsmittelabschnitte (2) flach ausgebildet ist, und wobei weiterhin jeder der Leitungsmittelabschnitte (2) mit Ausnahme der beiden endständigen Öffnungen (5) über keine weiteren Öffnungen verfügt,
c) optional Bereitstellen eines Verbindungselementes (8), an der die Endabschnitte der mindestens drei Leitungsmittelabschnitte (2) unlösbar befestigt werden können und welches auf die zweite Seite der ersten flexiblen Folie (1) vorzugsweise mittig aufgebracht werden kann,
d) optional Verbinden der Endabschnitte der mindestens drei Leitungsmittelabschnitte (2) mit dem Verbindungselement (8),
e) Aufbringen der mindestens drei Leitungsmittelabschnitte (2) oder des Verbundes aus den Leitungsmittelabschnitten und dem Verbindungselement (8) auf die zweite Seite der ersten Folie (1), wobei eine kreuz- oder sternförmige Anordnung der Leitungsmittelabschnitte (2) auf der ersten Folie (1) bevorzugt ist,
f) optional Aufbringen einer zweiten flexiblen Folie (4) auf den Verbund aus erster Folie (1) und Leitungsmittelabschnitten (2), wobei die zweite Folie auf die im Gebrauch von der Wunde weg weisenden Seite des Verbundes aufgebracht wird,
g) Aufbringen mindestens einer randständigen und vorwiegend zur Mitte der Wundauflage hin offenen Tasche (25), wobei die Tasche (25) vorzugsweise durch das Aufbringen weiterer Materialstücke, insbesondere Folienstücke, auf die zweite Seite der ersten Folie (1) oder - falls vorhanden - auf die im Gebrauch von der Wunde abgewandte Seite zweiten flexiblen Folie (4).

Bei der im Schritt (a) verwendeten ersten Folie (1) sollte die Summe der offenen Fläche der in Folie (1) vorhandenen Öffnungen (6) möglichst mindestens 0,1 % und höchstens 25 % der Flächenerstreckung, vorzugsweise mindestens 10 % und höchstens 22 % der Flächenerstreckung der Folie betragen.

Der Durchmesser der in der ersten Folie (1) vorhandenen Öffnungen (6) sollte mindestens 0,2 mm und höchstens 0,4 mm, insbesondere 0,3 mm betragen, wobei mindestens 150 und höchstens 300 Öffnungen pro cm² vorhanden sind. Vorteilhafterweise kann eine Folie mit einem Flächengewicht von mindestens 30 g / m² und höchstens 150 g / m², vorzugsweise mindestens 45 g / m² und höchstens 95 g / m² und insbesondere mindestens 55 g / m² und höchstens 65 g / m² als erste Folie (1) verwendet werden.

Die in der ersten Folie (1) vorhandenen Öffnungen (6) können durch Perforieren der Folie derart erzeugt werden, dass weitestgehend konische oder zylindrische Öffnungen entstehen und die Folie infolgedessen eine glatte Seite und eine der glatten Seite gegenüberliegende aufgeraute Seite aufweist. Alternativ können Öffnungen mit einer dreidimensionalen Struktur bereits während der Herstellung der Folie erzeugt werden.

Die Leitungsmittelabschnitte werden dann in Schritt (e) vorzugsweise auf die aufgeraute Seite aufgebracht, so dass die glatte Seite der ersten Folie (1) im Gebrauch der Wundauflage als Wundkontaktschicht dient.

Die in Schritt (b) verwendeten mindestens drei Leitungsmittelabschnitte (2) sollten aus einem Material gefertigt sein, welches eine Shore A Härte von mindestens 20 und höchstens 70, vorzugsweise eine Shore A Härte von mindestens 30 und höchstens 65, insbesondere eine Shore A Härte von mindestens 40 und höchstens 62 aufweist. Geeignete Materialien umfassen EVA, PU, PVC, PE, PET, PTFE, TPE, Silikon oder eine Mischung daraus.

Die Endabschnitte der mindestens drei Leitungsmittelabschnitte (2) können ggf. vor dem Aufbringen der Leitungsmittelabschnitte (2) auf die erste Folie (1) an einem Verbindungselement (8), beispielsweise an einer Kunststoffplatte, in kreuz- oder sternförmiger Anordnung unlösbar befestigt werden (Schritt d). Der hierdurch entstandene Verbund aus Leitungsmittelabschnitten und Verbindungselement (8) kann die beabsichtigte Ausrichtung der Leitungsmittelabschnitte bei der Aufbringung der Leitungsmittelabschnitte (2) auf die erste Folie (1) in Schritt (e) erleichtern. Die Befestigung der Leitungsmittelabschnitte am Verbindungselement kann beispielsweise durch Verkleben oder durch Verschweißen bewerkstelligt werden.

Die mindestens drei Leitungsmittelabschnitte (2) oder - falls vorhanden - der Verbund aus Leitungsmittelabschnitten und Verbindungselement (8) werden auf die zweite Seite der ersten Folie (1) aufgebracht und vorzugsweise unlösbar an der ersten Folie befestigt. Dies kann beispielsweise durch Verkleben oder durch Verschweißen erfolgen. Gemäß einer alternativen Ausführungsform können die Leitungsmittelabschnitte (2) oder - falls vorhanden - der Verbund aus Leitungsmittelabschnitten und Verbindungselement (8) auch durch eine zweite flexible Folie (4) auf der ersten Folie gehalten werden. Die zweite flexible Folie (4) wird dabei derart mit der zweiten Seite der ersten Folie verbunden, dass die Leitungsmittelabschnitte oder - falls vorhanden - der Verbund aus Leitungsmittelabschnitten und Verbindungselement (8) zwischen beiden Folien taschenförmig befestigt wird. Hierbei ist es vorteilhaft, wenn erste und zweite Folie durch eine Vielzahl punktförmiger Verklebungen oder Verschweißungen miteinander unlösbar verbunden werden.

### Testung der Drainagekapazität der Wundauflage

Die Testung einer erfindungsgemäßen Wundauflage, insbesondere die Messung der Drainagekapazität der Wundauflage, kann unter Verwendung einer Messvorrichtung (50) für Wundverbände erfolgen. Eine derartige Messvorrichtung ist in der vom Anmelder der vorliegenden Erfindung zeitgleich eingereichten deutschen Patentanmeldung "Vorrichtung zur Simulation einer Wunde am offenen Abdomen" beschrieben. Auf den Inhalt dieser Patentanmeldung wird hiermit Bezug genommen.

Die in der vorgenannten Patentanmeldung beschriebene und zur Testung der Drainagekapazität der Wundauflage geeignete Messvorrichtung (50) zur Simulation einer Wunde am offenen Abdomen umfasst eine Vielzahl mit einem ersten Fluid gefüllter erster Hohlkörper (51) mit flexibler Wandstruktur und einen zweiten Hohlkörper (52) mit einer flexiblen äußeren Hüllstruktur, wobei die äußere Hüllstruktur mindestens eine erste Öffnung (53) zum Simulieren einer Wunde aufweist. Die mit einem ersten Fluid gefüllten ersten Hohlkörper (51) liegen dabei im Inneren des zweiten Hohlkörpers (52) vor.

In Figur 5 ist eine vollständige Messvorrichtung (50) für Wundverbände mit angelegtem NPWT-Abdominalverband schematisch dargestellt. Der zweite Hohlkörper (52) weist eine transparenter Hülle auf. In den zweiten Hohlkörper (52) wurde eine Vielzahl von mit Wasser gefüllten ersten Hohlkörpers (51) eingebracht. Die äußere Hüllstruktur des zweiten Hohlkörpers (52) weist eine die Wunde simulierende erste Öffnung (53), sowie eine zweite Öffnung (54) auf, welche eine kontinuierliche Zufuhr einer Blutersatzlösung in das Innere des zweiten Hohlkörpers ermöglicht. Die innere Seite der Hülle des zweiten Hohlkörpers (52) kann optional mit einer zusätzlichen Lage eines flexiblen Materials (521) ausgekleidet werden, beispielsweise mit einer Schaumstoffmatte aus Polyurethan, welche eine Dicke von 1 cm aufweist. Weiterhin ist eine Fluidquelle (531) vorgesehen, so dass Blutersatzlösung über die Leitung (532) und über die zweite Öffnung (54) in das Innere des zweiten Hohlkörpers (52) geleitet werden kann. Die Fluidquelle (531) umfasst einen Vorratsbehälter mit Blutersatzlösung und eine daran gekoppelte Pumpe (in Figur 5 nicht dargestellt). Das Leitungsmittel (532) wird durch die zweite Öffnung (54) in das Inneren des zweiten Hohlkörpers (52) geführt. Alternativ kann das Leitungsmittel (532) an ein geeignetes Anschlussstück (in Figur 5 nicht dargestellt), welches an der zweiten Öffnung (54) vorgesehen ist, angeschlossen werden.

Die Messvorrichtung umfasst weiterhin ein NPWT-System (Unterdrucktherapieeinrichtung 41) mit einer Unterdruckquelle (533), einen Sammelkanister (534) zur Aufnahme des aus dem Inneren des zweiten Hohlkörpers (52) abgesaugten Fluids und einer Drainageleitung (535), die zum Anschluss an das Unterdruckanschlussstück (Port; 536) eines

Unterdrucktherapieverbandes vorgesehen ist. Der in Figur 5 dargestellte Abdominalverband umfasst einen Folienanteil (537), eine Schaumstofflage (538), eine Abdeckfolie (539) und ein Unterdruckanschlussstück (536). Der Folienanteil (537) des Verbands liegt dem künstlichen Wundgrund (gebildet durch die ersten Hohlkörper (51)) im Sinne einer Wundkontaktschicht direkt auf. Der Folienanteil (537) wird in den durch Bauchdecke (simuliert durch den zweiten Hohlkörper (52)) und Gedärme (simuliert durch den ersten Hohlkörpers (51)) gebildeten Zwischenraum eingeschoben. Zur Erhöhung der Drainagekapazität des Verbandes können auf den Folienanteil (537) Leitungsmittelabschnitte, vorzugsweise in sternförmiger Anordnung, aufgebracht werden (in Figur 5 nicht dargestellt). Der Abdominalverband umfasst weiterhin eine Schaumstofflage (538) aus einem offenzelligen Polyurethanschaumstoff. Umfang und Dicke des Schaumstoffs (538) sollten möglichst exakt an die künstliche Wunde (erste Öffnung 3 im zweiten Hohlkörpers (52)) angepasst werden, so dass Schaumstoffränder und Wundränder idealerweise auf Stoss zu liegen kommen. Über die künstliche Wunde (53) wird eine luftdichte selbstklebende Abdeckfolie (539) aus einem Polyurethanfilm platziert und außerhalb des Bereichs der künstlichen Wunde an der Oberfläche des zweiten Hohlkörpers (52) fixiert. Die Messvorrichtung (540) dient zur Ermittlung des aus dem Inneren des zweiten Hohlkörpers (52) abgesaugten Fluidvolumens. Vorzugsweise handelt es sich bei der es sich bei der Messvorrichtung (540) um eine elektronische Waage. Das Fluidvolumen kann in diesem Falle anhand von Gewichtsdifferenzen berechnet werden. Idealerweise weist die Messvorrichtung (540) einen Computeranschluss auf, so dass die Messwerte während des Simulationsexperimentes kontinuierlich aufgezeichnet werden können. Weiterhin ist optional eine dritte Öffnung (55) am zweiten Hohlkörper (52) vorgesehen. Ober diese Öffnung können weitere Messgeräte, beispielsweise ein Druckmesser oder ein Temperatursensor in das Innere des zweiten Hohlkörpers (52) eingeführt werden. Optional können zusätzliche Drucksensoren im NPWT-Verband vorgesehen sein (in Figur 5 nicht dargestellt).

Durch eine optional vorhandene vierte Öffnung (56) können bei Bedarf Heiz- und/oder Kühlmittel (in Figur 5 nicht dargestellt) in das Innere des zweiten Hohlkörpers (52) geführt werden, um die Innentemperatur des Wundsimulators auf einen gewünschten Wert einzustellen.

Die mit einem ersten Fluid gefüllten ersten Hohlkörper (51) können optional in Ihrer Gesamtheit in eine zusätzliche sackartige Umhüllung eingebracht werden, wobei die zusätzliche Umhüllung vorzugsweise aus einem flexiblen Folienmaterial mit einer Dicke von ca. 0,5 bis 1,0 mm besteht (in Figur 5 nicht dargestellt). Im Bereich der künstlichen Wunde (53) ergibt sich durch die in ihrer Gesamtheit mit einer zusätzliche Umhüllung umfassten Hohlkörper (51) eine Oberflächenbeschaffenheit, die hinsichtlich Relief und Festigkeit einer realen Wunde im Abdominalbereich vergleichbar ist.

### Anwendung der Wundauflage

Bei der Anwendung zur Unterdrucktherapie großflächiger Wunden im Abdominalbereich wird zunächst die erfindungsgemäße Wundauflage (10) auf den Wundgrund (13) gelegt. Die Randbereiche der Wundauflage (10) können dann über eine Tiefe von ca. 1 bis 30 cm zwischen Bauchdecke (14) und Wundgrund (13) eingeschoben werden, um eine flächige Abdeckung des Wundgrundes (13) zu erreichen. Vorwiegend zur Mitte der Wundauflage (10) hin offene Taschen (25) können das Einschieben des Randbereichs der Wundauflage (10) zwischen Bauchdecke (14) und Wundgrund (13) erleichtern, indem der behandelnde Arzt einen Bauch- oder Darmspatel oder gegebenenfalls einen Finger in die Taschen einführt und sodann den Randbereich der Wundauflage zwischen Bauchdecke und den Wundgrund einbringt.

Vorteilhafterweise umfasst die Wundauflage (10) eine oder mehrere flüssigkeitsdurchlässige Schichten (11). Die eine oder mehreren flüssigkeitsdurchlässigen Schichten können einen porösen Polymerschaumstoff umfassen. Dabei ist es für die Wundheilung sehr förderlich, wenn die flüssigkeitsdurchlässige Schicht (11) dergestalt an die Form der Wunde angepasst wird, dass die Wundränder (15) in einem vollständigen Kontakt mit der einen oder mehreren flüssigkeitsdurchlässigen Schicht (11) stehen.

Zum luftdichten Verschließen des Wundbereichs wird ein luftundurchlässiges Abdeckmaterial (16) über die Wunde gelegt. Die Ränder des Abdeckmaterials (16) werden auf die intakte Haut geklebt. Des Weiteren wird ein Unterschlussanschlussstück (18) angebracht, um eine funktionelle Verbindung des Wundraums mit einer außerhalb des Abdeckmaterials (16) befindlichen Unterdruckquelle (21), beispielsweise einer Unterdruckpumpe, herzustellen, so dass ein Unterdruck im Wundraum herstellbar ist und Flüssigkeiten aus dem Wundraum absaugbar sind. Das Unterdruckanschlussstück (18) wird vorzugsweise auf die von der Wunde abgewandte Außenseite des Abdeckmaterials (16) aufgeklebt, wobei vor dem Aufkleben in das ansonsten luftundurchlässige Abdeckmaterial (16) eine geeignete Öffnung (17) geschnitten wird. Die Unterdrucktherapie wird eingeleitet, indem das Unterdruckanschlussstück (18) mit einer Unterdruckquelle (21) verbunden wird und ein konstanter Unterdruck oder ein zeitlich variierenden Unterdruck für eine Dauer von einigen Minuten bis zu mehreren Tagen angelegt wird.

Unter "konstantem Unterdruck" wird hier verstanden, dass der Unterdruck während der Behandlung im Wesentlichen konstant gehalten wird. "Im Wesentlichen konstant" bedeutet, dass während der Behandlung geringfügige Veränderungen des Unterdrucks, beispielsweise um 15 % nach oben oder unten, auftreten können.

Ein bevorzugter konstanter Unterdruck ist der Bereich von mindestens 80 mm Hg bis höchstens 250 mm Hg, vorzugsweise 125 mm Hg.

Unter "zeitlich variierendem Unterdruck" wird hier verstanden, dass der Unterdruck während der Behandlung gezielt variiert wird. Unter der gezielten Variation des Luftdrucks werden diejenigen Variationen des Luftdrucks verstanden, die einsetzen, wenn nach Anlegen des Unterdruckverbandes ein erster Sollwert für den Unterdruck erreicht wurde. Dagegen ist die erste Anstiegsphase des Unterdrucks, die nach dem Anlegen des Verbandes bis zum Erreichen des ersten Sollwertes auftritt, nicht von dem Begriff "zeitlich variierender Unterdruck" umfasst. Dies gilt analog für die am Ende der Behandlung nötige Absenkung des Luftdrucks auf den Umgebungsluftdruck, die gleichfalls nicht von dem Begriff "zeitlich variierender Unterdruck" umfasst ist.

Entsprechend wird mit der vorliegenden Erfindung ein Verfahren zur Unterdrucktherapie einer Wunde, insbesondere einer Wunde im Abdominalbereich, beschrieben, umfassend die Schritte
a) Auflegen einer erfindungsgemäßen Wundauflage nach einem der Ansprüche 1 bis 20 auf den Wundgrund
b) Abdichtung der Wunde unter Verwendung einer geeigneten luftdichten Abdeckung 16,
c) Anbringen eines Unterdruckanschlussmittels (18),
d) Anlegen von Unterdruck für mindestens 30 Minuten und für höchstens 5 Tage.

### Ausführungsbeispiel - Messung der Drainagekapazität von Wundauflagen

Die Messung der Drainagekapazität von Wundauflagen erfolgte mit einer wie vorstehend beschrieben Messvorrichtung zur Simulation einer Wunde am offenen Abdomen. Die Messvorrichtung umfasst einen Ball aus PVC und eine Vielzahl von mit Wasser gefüllten Hohlkörpern aus Gummi. Die mit Wasser gefüllten Hohlkörper wurden im Inneren des PVC-Balls in ihrer Gesamtheit in eine zusätzliche Hülle aus Polyethylen eingebracht. Eine das Innere des PVC-Balls auskleidende zusätzliche Lage eines flexiblen Materials (siehe 521 in Fig. 5) war nicht vorhanden. Der Ball weist eine erste Öffnung zur Simulation einer künstlichen Wunde und eine zweite Öffnung zur kontinuierlichen Zufuhr einer Blutersatzlösung auf. Der Unterdruckverband mit der zu testenden Wundauflage wurde an eine Unterdruck-Therapievorrichtung angeschlossen. Ein Anteil der Blutersatzlösung wurde vor Versuchsbeginn und vor dem Anlegen des Unterdruckverbandes über die erste Öffnung direkt in den PVC-Ball gegossen. Ein weiterer Anteil der Blutersatzlösung wurde während des Versuchs über die zweite Öffnung kontinuierlich mittels einer Pumpe zugeführt. Die Menge der während des Versuch mittels der Unterdruck-Therapievorrichtung abgesaugt Blutersatzlösung wurde durch Wiegen bestimmt. Des Weiteren wurde die Menge der nach dem Versuch im Unterdruckverband (Abdominalwundauflage und zusätzliche Schaumlage) vorhandenen Blutersatzlösung durch Wiegen bestimmt.

In einen transparenten Ball aus PVC mit einem Volumen von 15.187 cm³ und mit einer Wandstärke von 1,6 mm wurde zur Simulation einer Wunde ein ellipsenförmiges Loch mit einer offenen Fläche von etwa 295 cm² geschnitten. Der Ball wurde so gedreht, dass die derart hergestellte künstliche Wunde (erste Öffnung) nach oben zeigte.

In ca. 50 Hohlkörper aus Gummi und mit einer Wandstärke von 0,3 mm wurde jeweils ca. 268 ml Wasser gefüllt. Die Hohlkörper wurden Fluid-dicht verschlossen.

Ein Vakuumbeutel aus Polyethylen mit einer Wandstärke von ca. 1 mm wurde über die ellipsenförmige erste Öffnung des Balls in das Innere des PVC-Balls eingebracht. Der Vakuumbeutel war so dimensioniert, dass der gesamte Innenraum des PVC-Balls damit ausgekleidet werden konnte. Die mit Wasser gefüllten Hohlkörper aus Gummi wurden in ihrer Gesamtheit über die erste Öffnung des PVC-Balls in den im Inneren des PVC-Balls befindlichen Vakuumbeutel gelegt, d.h. die Hohlkörper aus Gummi befanden sich sowohl im Inneren des PVC-Balls als auch innerhalb des Vakuumbeutels als zusätzliche Hülle. Die im Vakuumbeutel befindlichen und mit Wasser gefüllten Hohlkörper aus Gummi füllten den inneren Hohlraum PVC-Balls weitestgehend aus. Der mit Hohlkörpern aus Gummi gefüllte Vakuumbeutel wurde durch Umfalten seines Randes verschlossen.

Zur Herstellung eines Liters der Blutersatzlösung wurden 566 g demineralisiertes Wasser, 425 g Glycerin, 9 g NaCl und 0,2 g des Farbstoffs Allura Red vermischt. Die Lösung weist bei 23°C eine Viskosität von ca. 4,5 mPa*s auf.

Vor Versuchsbeginn und vor dem Anlegen des Unterdruckverbandes wurde über die erste Öffnung 250 ml Blutersatzlösung (Vorfüllvolumen) direkt in den PVC-Ball gegossen.

Die zu testenden Abdominalwundauflagen wurden wie folgt in die künstliche Wunde appliziert:
Versuch 1 mit Wundauflage 1: Wundauflage ABThera^{™} des Herstellers KCI (USA). Das Grundelement der ABThera^{™} Wundauflage ist eine geschlitzte Polyurethanfolie. Auf die Folie sind in sternförmiger Anordnung segmentierte Schaumstoffstreifen aufgebracht. Auf der im Gebrauch von der Wunde abgewandten Seite der Anordnung aus Folie und
Schaumstoffstreifen befindet sich eine weitere Lage einer geschlitzten Folie. Im zentralen Bereich der Wundauflage wurde eine weitere Schaumstofflage aus Polyurethan (VivanoMed^{®}-Schaum der Firma Paul Hartmann AG, Heidenheim, Deutschland) derart aufgelegt, dass die Ränder der weiteren Schaumstofflage in direktem Kontakt, d.h. auf Stoss, mit den Rändern der künstlichen Wunde (erste Öffnung, welche in den PVC-Ball geschnitten wurde) stehen. Die künstliche Wunde wurde mit einer luftdichten Abdeckfolie abgedeckt, wobei die Abdeckfolie auf die äußere Oberfläche des PVC-Balls in der Umgebung der künstlichen Wunde geklebt wurde. Über ein Unterdruckanschlussstück (Port) wurde eine Unterdruckkommunikation zwischen dem Wundraum der künstlichen Wunde und
einer Unterdruckquelle ATMOS S041 (Atmos, Deutschland) hergestellt.

Versuch 2 mit Wundauflage 2: Prototyp für eine erfindungsgemäße Abdominalwundauflage. Auf eine gelochte Folie aus Polyolefin (Medifol ^{®} 3D von rkw ProLife, Wasserburg, Deutschland) mit insgesamt runder Form wurden 6 Flachdrainage-Schläuche (Primed, Deutschland) aufgebracht. Die Befestigung der Flachdrainage-Schläuche auf der Folie erfolgte durch Verkleben mittels eines Klebstoffes auf Basis von Cyanoacrylat. Die kreisförmige Folie weist einen Durchmesser von 60 cm auf. Jeder der Leitungsmittelabschnitte (Abschnitt eines Flachdrainage-Schlauches) weist eine Länge von 27 cm, eine Breite (B) von ca. 2 cm und eine Dickenerstreckung (H) von ca. 0,53 cm auf. Jeder der Leitungsmittelabschnitte weist zwei durchgehende, nicht miteinander verbundene Hohlräume auf. Der Querschnitt des Leitungsmittelabschnittes ist in Fig. 6 schematisch gezeigt. Die Shore A Härte des Leitungsmittelabschnittes beträgt 60. Der Leitungsmittelabschnitt weist auf der Innenseite Vorwölbungen bzw. Verdickungen auf, die der Stabilisierung des Leitungsmittelabschnitts im Unterdruck dienen. Die Wandstärke des Leitungsmittelabschnittes beträgt an der dünnsten Stelle 0,7 mm. Jeder der Leitungsmittelabschnitte verfügt mit Ausnahme der endständigen Öffnungen über keine weiteren Öffnungen. Die Leitungsmittelabschnitte sind in sternförmiger Anordnung auf die gelochte Folie aufgebracht, wobei die sich die Leitungsmittelabschnitte bis zum Rand der Folie erstrecken. Die zur Mitte der Wundauflage hin orientierten Enden der Leitungsmittelabschnitte sind voneinander beabstandet. Der Abstand a (siehe Fig. 1 a, Ausschnitt) der Enden zweier gegenüberliegender Leitungsmittelabschnitte beträgt ca. 6 cm.

Die Summe der offenen Fläche der in der ersten Folie (1) vorhandenen Öffnungen (6) beträgt 14 % der Flächenerstreckung der Folie. Der Durchmesser der in der ersten Folie (1) vorhandenen Öffnungen (6) beträgt 0,3 mm, wobei ca. 168 Öffnungen (6) pro cm² vorhanden sind. Die Folie weist ein Flächengewicht von 60 g / m² auf, wobei das Flächengewicht nach der Norm EN ISO 2286 - 2 bestimmt wird. Die Zugfestigkeit der Folie, bestimmt nach EN ISO 527, beträgt (MD / CD) 22 / 17 N/inch. Die Bruchdehnung der Folie, bestimmt nach EN ISO 527, beträgt (MD / CD) 500 / 500 %. Die Polyolefin-Folie weist trichterförmige bzw. konisch geformte Öffnungen auf, die über eine dreidimensionale Struktur verfügen, so dass die Folie infolgedessen eine glatte Seite und eine der glatten Seite gegenüberliegende aufgeraute Seite aufweist. Die glatte Seite ist zum Aufbringen auf den künstlichen Wundgrund vorgesehen, während die Leitungsmittelabschnitte auf die aufgeraute Seite aufgebracht werden.

Im zentralen Bereich der Wundauflage wurde, wie bei Wundauflage 1 beschrieben, eine weitere Schaumstofflage aus Polyurethan (VivanoMed-^{®} Schaum der Firma Paul Hartmann AG, Heidenheim, Deutschland) derart aufgebracht, dass die Ränder der weiteren Schaumstofflage in direktem Kontakt, d.h. auf Stoss, mit den Rändern der künstlichen Wunde (Öffnung, welche in den PVC-Ball geschnitten wurde) stehen. Die luftdichte Abdeckung der künstlichen Wunde und der Anschluss an eine Unterdruckquelle erfolgten wie vorstehend unter Wundauflage 1 beschrieben.

Die Versuchsdauer betrug jeweils 60 min. Es wurde ein konstanter Unterdruck von 125 mm Hg angelegt.

Über eine an die zweite Öffnung (siehe 54 in Fig. 5) des Balls angeschlossene Leitung wurde über den Versuchszeitraum von 60 min insgesamt ca. 500 ml einer gefärbten Blutersatzlösung kontinuierlich und mit gleich bleibender Flussrate eingebracht (Exsudatzufluss).

Der Versuch wurde bei Raumtemperatur durchgeführt (23°C). Eine Temperierung der Blutersatzlösung erfolgte nicht, d.h. die Blutersatzlösung hatte gleichfalls eine Temperatur von etwa 23°C. Die über das NPWT-System abgesaugte Blutersatzlösung wurde im Kanister der Unterdruckeinheit gesammelt. Das Volumen der nach 60 min in den Kanister abgesaugten Blutersatzlösung wurde anhand der Gewichtsdifferenz zwischen leerem Kanister (vor Versuchsbeginn) und gefülltem Kanister (nach Versuchsdurchführung) berechnet. Des Weiteren wurde das Volumen der nach dem Versuch im Unterdruckverband (Abdominalwundauflage und zusätzliche Schaumlage) vorhandenen Blutersatzlösung anhand der Gewichtsdifferenz zwischen trockenem Unterdruckverband (vor Versuchsbeginn) und feuchtem Unterdruckverband (nach Versuchsdurchführung) berechnet. Das Volumen der im Wundsimulator (mit Hohlkörpern aus Gummi gefüllter PVC-Ball) verbliebenen Blutersatzlösung wurde berechnet:

| | Wundsimulator (ml) | Kanister (ml) | Wundauflage (ml) | Schaum (ml) | Summe (ml) |
|---|---|---|---|---|---|
| **Versuch 1** | 194,0 | 276,6 | 163,5 | 115,9 | 750,0 |
| **Versuch 2** | 152,8 | 455,2 | 25,5 | 116,5 | 750,0 |

Insgesamt wurde bei dem mit Wundauflage 2 durchgeführtem Experiment (Versuch 2) im Vergleich zu dem mit Wundauflagen 1 durchgeführten Experiment (Versuch 1) ein größeres Volumen an Blutersatzlösung in die Unterdruckquelle gesaugt (455,2 ml gegenüber 276,6 ml). Wundauflage 2 zeigt folglich unter den vorgenannten Versuchsbedingungen am Wundsimulator eine vergleichsweise höhere Drainagekapazität.

Bei Versuch 1 wurde ein größeres Volumen Blutersatzlösung in der Wundauflage zurückgehalten.

Anhand der Färbung der Blutersatzlösung konnte während des Versuchs beobachtet werden, dass die Blutersatzlösung bei beiden Wundauflagen 1 und 2 kaum in den durch die Schaumlage und Folienlage gebildeten Zwischenraum eindringt. Stattdessen scheint die Blutersatzlösung bevorzugt zwischen den künstlichen Gedärmen (Oberfläche der mit einer zusätzlichen Hülle umgebenen Hohlkörper aus Gummi) und der wundseitigen Oberfläche der geschlitzten Folie zum Zentrum der Wundauflage zu fließen und dort abgesaugt zu werden. Die im Vergleich zu Wundauflage 1 erhöhte Drainagekapazität der erfindungsgemäßen Wundauflage 2 könnte auf einem verbesserten Abfluss von im peripheren Bereich der künstlichen Wunde vorhandener Blutersatzlösung durch die offenen Lumina der Leitungsmittelabschnitte beruhen.

## Patentansprüche

1. Wundauflage zur Verwendung bei der Unterdrucktherapie abdominaler Wunden, umfassend
i) eine erste flexible Folie (1) mit einer ersten und einer zweiten Seite, wobei die erste Seite zum Aufbringen auf den Wundgrund (13) vorgesehen ist, und wobei die erste Folie (1) eine Vielzahl von über die Fläche verteilte Öffnungen (6) aufweist,
ii) mindestens drei auf der zweiten Seite der ersten Folie (1) aufgebrachte Leitungsmittelabschnitte (2) aus einem biegsamen elastomeren Material mit einer Dickenerstreckung (H) von höchstens 20 mm,
wobei jeder der Leitungsmittelabschnitte (2) mindestens einen durchgehenden Hohlraum (3) aufweist,
wobei jeder der Leitungsmittelabschnitte (2) flach ausgebildet ist,
und wobei jeder der Leitungsmittelabschnitte (2) mit Ausnahme der endständigen Öffnungen (5) keine weiteren Öffnungen aufweist,
**dadurch gekennzeichnet,**
**dass** die Wundauflage auf der im Gebrauch von der Wunde abgewandten Seite mindestens eine zur Mitte der Wundauflage hin offene Tasche (25) aufweist, welche das gleichmäßige Aufbringen und Auslegen der Wundauflage auf den Wundgrund (13) erleichtert.

2. Wundauflage nach Anspruch 1, wobei die Leitungsmittelabschnitte (2) mit wenigstens 25 % ihrer senkrecht auf die erste Folie (1) projizierten Fläche mit der zweiten Seite der ersten Folie (1) unlösbar und flächenhaft verbunden sind.

3. Wundauflage nach einem der vorangehenden Ansprüche, wobei die Leitungsmittelabschnitte (2) aus einem Material gefertigt sind, welches eine Shore A Härte von mindestens 20 und höchstens 70 (gemessen nach DIN 53505 vom August 2000, bei einer Temperatur von 23°C) aufweist und wobei der Drainageschlauch weiterhin EVA, PU, PVC, PE, PET, PTFE, TPE, Silikon oder eine Mischung daraus umfassen kann.

4. Wundauflage nach einem der vorangehenden Ansprüche, wobei es sich bei den Leitungsmittelabschnitten (2) um Abschnitte eines ein- oder mehrlumigen Drainageschlauches handelt, und wobei die Leitungsmittelabschnitte (2) durch Ablängen aus einem längerem Drainageschlauch herstellbar sind.

5. Wundauflage nach einem der vorangehenden Ansprüche, wobei die Summe der offenen Fläche der in der ersten Folie (1) vorhandenen Öffnungen (6) mindestens 0,1 % und höchstens 25 % der Flächenerstreckung der Folie beträgt.

6. Wundauflage nach einem der vorangehenden Ansprüche, wobei der Durchmesser der in der ersten Folie (1) vorhandenen Öffnungen (6) mindestens 0,2 mm und höchstens 0,4 mm beträgt.

7. Wundauflage nach einem der vorangehenden Ansprüche, wobei die Anzahl der in der ersten Folie (1) pro Fläche vorhandenen Öffnungen (6) mindestens 150 pro cm² und höchstens 300 pro cm² beträgt.

8. Wundauflage nach einem der vorangehenden Ansprüche, wobei das Flächengewicht der ersten Folie (1) mindestens 30 g / m² und höchstens 150 g / m² beträgt.

9. Wundauflage nach einem der vorangehenden Ansprüche, wobei die in der ersten Folie (1) vorhandenen Öffnungen (6) eine weitestgehend konische oder zylindrische dreidimensionale Form aufweisen und die Folie infolgedessen eine glatte Seite und eine der glatten Seite gegenüberliegende aufgeraute Seite aufweist.

10. Wundauflage nach Anspruch 9, wobei die zum Aufbringen auf den Wundgrund (13) vorgesehene erste Seite der ersten Folie (1) durch die glatte Seite der ersten Folie (1) gebildet wird und die zweite Seite der ersten Folie (1) durch die aufgeraute Seite gebildet wird.

11. Wundauflage nach einem der vorangehenden Ansprüche, wobei die Leitungsmittelabschnitte auf der zweiten Seite der ersten Folie (1) voneinander getrennt vorliegen, dergestalt dass kein die vorhandenen Leitungsmittelabschnitte Fluid-leitend verbindender kontinuierlicher Hohlraum gebildet wird.

12. Wundauflage nach einem der vorangehenden Ansprüche, weiterhin umfassend eine zweite flexible Folie (4), wobei die zweite Folie (4) auf die im Gebrauch von der Wunde abgewandte Seite des Verbundes aus erster Folie (1) und Leitungsmittelabschnitten (2) unlösbar aufgebracht ist.

13. Wundauflage nach Anspruch 12, wobei die zweite Folie (4) eine Vielzahl von über die Fläche verteilten Öffnungen aufweist, welche zum Durchleiten von Fluid geeignet sind oder wobei die zweite Folie weitestgehend Fluid-undurchlässig ausgebildet ist und lediglich eine einzige Öffnung (10) aufweist.

14. Wundauflage nach einem der vorangehenden Ansprüche, wobei es sich bei der ersten flexiblen Folie (1) und/oder - falls vorhanden - bei der zweiten flexiblen Folie (4) um eine thermoplastische Folie handelt.

15. Wundauflage nach einem der vorangehenden Ansprüche, weiterhin umfassend ein Verbindungselement (8), welches auf die zweite Seite der ersten flexiblen Folie (1) aufgebracht werden kann und an dem die Endabschnitte der mindestens drei Leitungsmittelabschnitte (2) unlösbar befestigt werden können.

16. Wundauflage nach einem der vorangehenden Ansprüche, weiterhin umfassend eine oder mehrere flüssigkeitsdurchlässige Schichten (11), zum Aufbringen auf die im Gebrauch von der Wunde abgewandte Seite der Wundauflage.

17. Verband zur Verwendung bei der Unterdrucktherapie von Wunden, umfassend eine Wundauflage nach einem der vorangehenden Ansprüche, weiterhin umfassend ein luftundurchlässiges Abdeckmaterial (16) zum luftdichten Verschließen der Wunde und der Wundumgebung.

18. Vorrichtung zur Verwendung bei der Unterdrucktherapie von Wunden, umfassend einen Verband nach Anspruch 17,
weiterhin umfassend ein auf das luftundurchlässige Abdeckmaterial (16) auf der von der Wunde abgewandten Seite aufbringbares Mittel (18) zur funktionellen Verbindung des Wundraums mit einer außerhalb des Abdeckmaterials (16) befindlichen Unterdruckquelle (21), so dass ein Unterdruck im Wundraum herstellbar ist und Flüssigkeiten aus dem Wundraum absaugbar sind.

19. Gebrauchsfertiges Set zur Unterdruck-Wundbehandlung, umfassend
a) eine Wundauflage nach einem oder mehreren der vorangehenden Ansprüche 1 bis 15,
b) mindestens eine flüssigkeitsdurchlässige Schicht (11) zum Aufbringen auf die im Gebrauch von der Wunde abgewandte Seite der Wundauflage,
c) ein luftundurchlässiges Abdeckmaterial (16) zum luftdichten Verschließen der Wunde und der Wundumgebung,
d) ein Unterdruck-Anschlussmittel (18) zur funktionellen Verbindung des Wundraums mit einer außerhalb des Abdeckmaterials befindlichen Unterdruckquelle (21), so dass ein Unterdruck im Wundraum herstellbar ist und Flüssigkeiten aus dem Wundraum absaugbar sind,
und wobei die Komponenten a) bis d) steril verpackt vorliegen.

## Claims

1. Wound dressing for use in negative-pressure abdominal wound therapy, comprising
i) a first flexible film (1) with a first and a second side, with the first side being provided for application on the wound bed (13), and with the first film (1) having a multiplicity of openings (6) distributed across the area,
ii) at least three conduit sections (2), applied to the second side of the first film (1), made of a flexible elastomeric material with a thickness (H) of at most 20 mm,
with each of the conduit sections (2) having at least one continuous cavity (3),
with each of the conduit sections (2) having a flat design,
and, with the exception of the openings (5) situated at the ends, each of the conduit sections (2) having no further openings, **characterized in that** the wound dressing has at least one pocket (25) which is open toward the center of the wound dressing on the side that faces away from the wound during use and which simplifies the uniform application of the wound dressing to the wound bed (13) and the laying out of said wound dressing on the latter.

2. Wound dressing according to Claim 1, wherein the conduit sections (2) are connected in non-detachable and areal fashion to the second side of the first film (1) with at least 25 % of their area projected perpendicularly onto the first film (1).

3. Wound dressing according to one of the preceding claims, wherein the conduit sections (2) are made of a material which has a Shore A hardness of at least 20 and of at most 70 (measured according to DIN 53505 from August 2000, at a temperature of 23°C), and wherein the drainage tube can furthermore comprise EVA, PU, PVC, PE, PET, PTFE, TPE, silicone or a mixture thereof.

4. Wound dressing according to one of the preceding claims, wherein the conduit sections (2) are sections of a single-lumen or multi-lumen drainage tube and wherein the conduit sections (2) can be produced by cutting a longer drainage tube to length.

5. Wound dressing according to one of the preceding claims, wherein the sum of the open area of the openings (6) present in the first film (1) is at least 0.1 % and at most 25 % of the area extent of the film.

6. Wound dressing according to one of the preceding claims, wherein the diameter of the openings (6) present in the first film (1) is at least 0.2 mm and at most 0.4 mm.

7. Wound dressing according to one of the preceding claims, wherein the number of openings (6) present per unit area in the first film (1) is at least 150 per cm² and at most 300 per cm².

8. Wound dressing according to one of the preceding claims, wherein the mass per unit area of the first film (1) is at least 30 g / m² and at most 150 g / m².

9. Wound dressing according to one of the preceding claims, wherein the openings (6) present in the first film (1) have a conical or cylindrical three-dimensional shape to the greatest possible extent and, as a result of this, the film has a smooth side and a roughened side which lies opposite to the smooth side.

10. Wound dressing according to Claim 9, wherein the first side of the first film (1) provided for application on the wound bed (13) is formed by the smooth side of the first film (1) and the second side of the first film (1) is formed by the roughened side.

11. Wound dressing according to one of the preceding claims, wherein the conduit sections on the second side of the first film (1) are present separated from one another such that there is no formation of a continuous cavity that connects the present conduit sections in a fluid-conducting fashion.

12. Wound dressing according to one of the preceding claims, furthermore comprising a second flexible film (4), wherein the second film (4) is applied in a non-detachable fashion to the side of the composite of first film (1) and conduit sections (2) that faces away from the wound during use.

13. Wound dressing according to Claim 12, wherein the second film (4) has a multiplicity of openings distributed across the area thereof, which openings are suitable for passing fluids through them, or wherein the second film is embodied to be impermeable to fluid to the greatest possible extent and merely has a single opening (10).

14. Wound dressing according to one of the preceding claims, wherein the first flexible film (1) and/or - if present - the second flexible film (4) is a thermoplastic film.

15. Wound dressing according to one of the preceding claims, furthermore comprising a connection element (8), which can be applied to the second side of the first flexible film (1) and to which the end sections of the at least three conduit sections (2) can be attached in a non-detachable fashion.

16. Wound dressing according to one of the preceding claims, furthermore comprising one or more liquid-permeable layers (11), for application on the side of the wound dressing that faces away from the wound during use.

17. Bandage for use in negative-pressure wound therapy, comprising a wound dressing according to one of preceding claims, furthermore comprising an air-impermeable cover material (16) for airtight sealing of the wound and the surroundings of the wound.

18. Device for use in negative-pressure wound therapy, comprising a bandage according to Claim 17, furthermore comprising a means (18) which can be applied to the air-impermeable cover material (16) on the side that faces away from the wound, for the functional connection of the wound space to a negative-pressure source (21) situated outside of the cover material (16) such that negative pressure can be set in the wound space and liquids can be suctioned out of the wound space.

19. Ready-made set for negative-pressure wound therapy, comprising
a) a wound dressing according to one or more of preceding Claims 1 to 17,
b) at least one liquid-permeable layer (11) for application on the side of the wound dressing that faces away from the wound during use,
c) an air-impermeable cover material (16) for airtight closure of the wound and the surroundings of the wound,
d) a negative-pressure connection means (18) for the functional connection of the wound space to a negative-pressure source (21) situated outside of the cover material such that negative pressure can be set in the wound space and liquids can be suctioned out of the wound space,
and wherein components a) to d) are present in sterile packaged form.

## Revendications

1. Pansement à utiliser pour la thérapie de plaies abdominales par dépression, comprenant:
i) une première feuille souple (1) présentant un premier côté et un deuxième côté, dans lequel le premier côté est prévu pour être appliqué sur le fond de la plaie (13), et dans lequel la première feuille (1) présente une multiplicité d'ouvertures (6) réparties sur la surface,
ii) au moins trois parties de moyens de conduite (2) déposés sur le deuxième côté de la première feuille (1), constituées d'un matériau élastomère flexible ayant une dimension en épaisseur (H) de 20 mm au maximum,
dans lequel chacune des parties de moyens de conduite (2) présente au moins une cavité continue (3),
dans lequel chacune des parties de moyens de conduite (2) est plate,
et dans lequel chacune des parties de moyens de conduite (2) ne présente pas d'autres ouvertures, à l'exception des ouvertures aux extrémités (5), **caractérisé en ce que** le pansement présente, sur le côté situé à l'opposé de la plaie en position d'utilisation, au moins une poche (25) ouverte vers le milieu du pansement, qui facilite l'application et le placement uniformes du pansement sur le fond de la plaie (13).

2. Pansement selon la revendication 1, dans lequel les parties de moyens de conduite (2) sont assemblées à plat et de façon inséparable au deuxième côté de la première feuille (1) par au moins 25 % de leur surface projetée perpendiculairement sur la première feuille (1).

3. Pansement selon l'une quelconque des revendications précédentes, dans lequel les parties de moyens de conduite (2) sont fabriquées en un matériau, qui présente une dureté Shore A d'au moins 20 et d'au plus 70 (mesurée selon la norme DIN 53505 d'août 2000, à une température de 23°C) et dans lequel le tuyau de drainage peut en outre comprendre du EVA, PU, PVC, PE, PET, PTFE, TPE, du silicone ou un mélange de ceux-ci.

4. Pansement selon l'une quelconque des revendications précédentes, dans lequel les parties de moyens de conduite (2) sont des parties d'un tuyau de drainage à une ou plusieurs lumière(s), et dans lequel les parties de moyens de conduite (2) peuvent être produites par découpage à longueur d'un tuyau de drainage plus long.

5. Pansement selon l'une quelconque des revendications précédentes, dans lequel la somme des surfaces ouvertes des ouvertures (6) présentes dans la première feuille (1) vaut au moins 0,1 % et au plus 25 % de l'extension superficielle de la feuille.

6. Pansement selon l'une quelconque des revendications précédentes, dans lequel le diamètre des ouvertures (6) présentes dans la première feuille (1) vaut au moins 0,2 mm et au plus 0,4 mm.

7. Pansement selon l'une quelconque des revendications précédentes, dans lequel le nombre des ouvertures (6) présentes dans la première feuille (1) par unité de surface vaut au moins 150 par cm² et au plus 300 par cm².

8. Pansement selon l'une quelconque des revendications précédentes, dans lequel le grammage de la première feuille (1) vaut au moins 30 g/m² et au plus 150 g/m².

9. Pansement selon l'une quelconque des revendications précédentes, dans lequel les ouvertures (6) présentes dans la première feuille (1) présentent une forme tridimensionnelle très largement conique ou cylindrique et la feuille présente par conséquent un côté lisse et un côté rugueux à l'opposé du côté lisse.

10. Pansement selon la revendication 9, dans lequel le premier côté de la première feuille (1) prévu pour être appliqué sur le fond de la plaie (13) est formé par le côté lisse de la première feuille (1) et le deuxième côté de la première feuille (1) est formé par le côté rugueux.

11. Pansement selon l'une quelconque des revendications précédentes, dans lequel les parties de moyens de conduite sont séparées l'une de l'autre sur le deuxième côté de la première feuille (1), de telle manière qu'il ne soit formé aucune cavité continue reliant en connexion fluidique les parties de moyens de conduite présentes.

12. Pansement selon l'une quelconque des revendications précédentes, comprenant en outre une deuxième feuille souple (4), dans lequel la deuxième feuille (4) est déposée de façon inséparable sur le côté du composite formé par la première feuille (1) et les parties de moyens de conduite (2), qui est situé à l'opposé de la plaie dans la position d'utilisation.

13. Pansement selon la revendication 12, dans lequel la deuxième feuille (4) présente une multiplicité d'ouvertures réparties sur la surface, qui conviennent pour le passage de fluide ou dans lequel la deuxième feuille est très largement imperméable au fluide et présente uniquement une seule ouverture (10).

14. Pansement selon l'une quelconque des revendications précédentes, dans lequel la première feuille souple (1) et/ou - si elle est présente - la deuxième feuille souple (4) est une feuille thermoplastique.

15. Pansement selon l'une quelconque des revendications précédentes, comprenant en outre un élément de liaison (8), qui peut être déposé sur le deuxième côté de la première feuille souple (1) et sur lequel les parties d'extrémité desdites au moins trois parties de moyens de conduite (2) peuvent être fixées de façon inséparable.

16. Pansement selon l'une quelconque des revendications précédentes, comprenant en outre une ou plusieurs couche(s) (11) perméable(s) au liquide à appliquer sur le côté du pansement situé à l'opposé de la plaie dans la position d'utilisation.

17. Bandage à utiliser pour la thérapie de plaies par dépression, comprenant un pansement selon l'une quelconque des revendications précédentes, comprenant en outre un matériau de recouvrement imperméable à l'air (16) pour la fermeture hermétique de la plaie et des alentours de la plaie.

18. Dispositif à utiliser pour la thérapie de plaies par dépression, comprenant un bandage selon la revendication 17,
comprenant en outre un moyen (18) applicable sur le matériau de recouvrement imperméable à l'air (16) sur le côté situé à l'opposé de la plaie pour la liaison fonctionnelle de l'espace de la plaie avec une source de dépression (21) se trouvant à l'extérieur du matériau de recouvrement (16), de telle manière qu'une dépression puisse être produite dans l'espace de la plaie et que des liquides puissent être aspirés hors de l'espace de la plaie.

19. Ensemble prêt à l'emploi pour le traitement de plaies par dépression, comprenant
a) un pansement selon l'une quelconque des revendications 1 à 15,
b) au moins une couche perméable au liquide (11) à appliquer sur le côté du pansement situé à l'opposé de la plaie dans la position d'utilisation,
c) un matériau de recouvrement imperméable à l'air (16) pour la fermeture hermétique de la plaie et des alentours de la plaie,
d) un moyen de raccordement de dépression (18) pour la liaison fonctionnelle de l'espace de la plaie avec une source de dépression (21) se trouvant à l'extérieur du matériau de recouvrement, de telle manière qu'une dépression puisse être produite dans l'espace de la plaie et que des liquides puissent être aspirés hors de l'espace de la plaie,
et dans lequel les composants a) à d) se trouvent sous un emballage stérile.
